# EUROPEAN PATENT SPECIFICATION

(11) **EP 2 417 951 B1**
(45) Date of publication and mention of the grant of the patent: **14.12.2016**
(21) Application number: 11177496.4
(22) Date of filing: 12.08.2011
(51) Int. Cl.: A61F 13/472, A61F 13/533, A61F 13/15

(54) **Method and apparatus for making a fibrous article having a three dimensional profile and an absorbant article including a formed fibrous article**
Verfahren und Vorrichtung zur Herstellung eines faserigen Artikels mit dreidimensionalem Profil und aufnahmefähiger Artikel mit dem geformten faserigen Artikel
Procédé et appareil pour fabriquer un article fibreux doté d'un profil tridimensionnel et article absorbant incluant un article fibreux formé

(30) Priority: 12.08.2010 US 855175; 12.08.2010 US 855183; 18.08.2010 US 858619
(43) Date of publication of application: 15.02.2012
(73) Proprietor: Johnson & Johnson do Brasil Industria e Comercio de Produtos Para Saude Ltda., CEP 12237-350 Sao Paulo (BR)
(72) Inventor: Alkmin, Marco Antonio, 12232-090 Sao Paulo (BR); Cau, Jose Francisco, 12242-170 Sao Paulo (BR); Coutinho, Jose Manoel Soares, Sao Paulo (BR); Hernandez, Francisco J. V., 12242-530 Sao Paulo (BR); Rimoli, Francisco Antonio, 08735-420 Sao Paulo (BR); Neto, Francisco Savastano, Sao Paulo (BR); Duarte, Ivair Luiz, Sao Paulo (BR); Faria, Reinaldo Lourenco, 12237-160 Sao Paulo (BR); Yamashita, Alexandre Teixeira, 12242-460 Sao Paulo (BR); Barbosa, Livea Fujita, 12243-840 Sao Paulo (BR); Martines, Manuela Leonel, Sao Paulo (BR)
(74) Representative: Kirsch, Susan Edith

(56) References cited:
- EP-A1- 0 733 729
- EP-A1- 2 085 504
- DE-A1- 2 113 508
- GB-A- 2 202 554
- US-A1- 2010 036 343

## Description

### FIELD OF THE INVENTION

The present invention generally relates to a method and apparatus for a making a formed fibrous article and more specifically to method and apparatus for making a formed fibrous article useful as an absorbent core structure in a disposable sanitary article such as a sanitary napkin, panty liner, diaper or the like. Also disclosed is a disposable sanitary article including a formed fibrous article as a core structure thereof.

### BACKGROUND OF THE INVENTION

Various methods for making formed fibrous articles from fibrous materials such as cellulose or the like are well know to those of skill in the art. One common method of manufacturing such formed fibrous articles consists of defiberizing a starting material and then creating an air-entrained stream of the defiberized material. The air-entrained defiberized material may be formed into a formed fibrous article through the use of a porous mold structure subjected to a vacuum to draw the defiberized material into the mold.

It is also known that formed fibrous articles of the type described above may be subjected to calendering processes to alter the mechanical and fluid handling properties of such articles. A calendering process used in the art is commonly referred to as "pin calendering". Pin calendering employs the use of a plurality of pins adapted to compress and densify the article.

A problem associated with pin calendering processes of the type described above is that such processes typically require that a "carrier layer" be used during the pin-calendering step. "Carrier layer" as used herein means any material layer used to support the formed fibrous article, such as a conveyer belt or an adjacent material layer such as rolled nonwoven layer or the like. The use of a carrier layer increases complexity of manufacture. In addition, if the carrier layer is a layer intended to be incorporated into the final product, the inclusion of such layer may increase the cost of the final product and/or undesirably affect the fluid handling characteristics of the product.

In view of the above the inventors of the present invention have discovered, and disclosed herein, a method and apparatus for making a pin-calendered formed fibrous article that does not require the use of a carrier layer. Formed fibrous articles according to the present invention are particularly useful as an absorbent core structure in a disposable sanitary article such as a sanitary napkin, panty liner, diaper or the like.

US 2010/036343 (A1) concerns an absorbent article including a laminate structure including cover, transfer layer, core and barrier, the absorbent core having a first central region and second region concentrically surrounding the first region.

EP 0733729 (A1) concerns a mechanism to transfer a fibre web between a conveyor belt and two calender rollers, wherein the mechanism has a suction cylinder with a fixed vacuum zone which rotates in the direction of the conveyor belt movement and against the rotation of the lower calender roller.

EP 2085504 (A1) concerns an apparatus used for transferring a nonwoven web between an upstream unit, such as for example a conveyor belt, and a downstream unit, such as for example a thermal bonding unit, that comprises a rotating and air permeable cylinder, and air suction means for creating a suction force through the cylinder in order to make a nonwowen web adhere against the surface of a first portion of the cylinder.

DE 2113508 (A1) concerns a pad for absorbing liquids excreted from the human body, e.g. a sanitary towel. The pad has an absorbent core formed of fibrous matter. The fibrous matter is compressed in the region where, in use, the pad receives the excreted liquid and the compressed region purports to serve to transfer the liquid to the remainder of the pad.

### SUMMARY OF THE INVENTION

In view of the foregoing, the present invention provides a method for making a formed fibrous article including the steps of forming a formed fibrous article having a three dimensional profile by forming a formed fibrous article and passing the formed fibrous article through a calendering station to thereby provide the formed fibrous article with the three dimensional profile; wherein the calendering station includes a vacuum roll and an opposed calender roll and a surface of the calender roll includes at least one recess, the recess being structured and arranged to provide the formed fibrous article with the three dimensional profile, pin-calendering the fibrous article in a pin calendering station between a first roll and a second roll, wherein the fibrous article is conveyed through a nip defined between the first roll and the second roll without the use of a carrier layer, wherein the first roll of the pin calendering station comprises a vacuum roll and the second roll comprises a pin calender roll.

The present invention also provides an apparatus for making a formed fibrous article having a three dimensional profile, the apparatus including a calendering station including a first roll and a second roll, wherein the second roll includes at least one recess for providing a formed fibrous article with a three dimensional profile, a pin calendering station including a first roll and a second roll, wherein the first roll and second roll are structured and arranged to convey a fibrous article through a nip defined between the first and second roll without the use of a carrier layer, wherein the first roll of the pin calendering station comprises a vacuum roll and the second roll comprises a pin calender roll.

### BRIEF DESCRIPTION OF THE DRAWINGS

Examples of embodiments of the present invention will now be described with reference to the drawings, in which:
Fig. 1 is a side elevation view of an apparatus according to the present invention;
Fig. 2 is a detailed perspective view of the forming drum that forms part of the apparatus according to the present invention;
Fig. 3 is a sectional view taken along line 3-3 in Fig. 2;
Fig. 4 is a detailed perspective view of a portion of the forming drum shown in Fig. 2;
Fig. 5 is a side elevation view of the forming drum and a calendering station that form part of the apparatus according to the present invention;
Fig. 6 is a detailed perspective view of the calendering station shown in Fig. 5, depicting the vacuum roll and calender roll thereof;
Fig. 7 is a elevation view of the that portion of the calendering station calender roll circled in Fig. 6;
Fig. 8 is a sectional view taken along line 8-8 in Fig. 6;
Fig. 9 is a detailed perspective view of that portion of the calendering station vacuum roll circled in Fig. 6;
Fig. 10 is a perspective view of a formed fibrous article after passing through the calendering station;
Fig. 11 is a side elevation view of a pin calendering station that forms part of the apparatus according to the present invention;
Fig. 12 is a detailed perspective view of the pin calendering station shown in Fig. 11, depicting the vacuum roll and pin calender roll thereof;
Fig. 13 is a partially exploded perspective view of the pin calendering station pin calender roll;
Fig. 14 is a detailed plan view of that portion of the pin calender roll circled in Fig. 13;
Fig. 15 is a sectional view taken along line 15-15 in Fig. 14;
Fig. 16 is detailed perspective view of that portion of the pin calender roll circled in Fig. 13;
Fig. 17 is a sectional view taken along line 17-17 in Fig. 11;
Fig. 18 is a sectional view taken along line 18-18 in Fig. 12;
Fig. 19 is a side elevation view of a transfer wheel that forms part of the apparatus according to the present invention;
Fig. 20 is a perspective view of the transfer wheel shown in Fig. 19;
Fig. 21 is a top plan view of the formed fibrous article after passing through the pin calendering station;
Fig. 22 is a detailed perspective view of that portion of the formed fibrous article circled in Fig. 21;
Figs. 23 is a perspective view of an absorbent article with the cover layer and transfer layer thereof partially broken away;
Fig. 24 is a sectional view taken along line 24-24 in Fig. 23; and
Figs. 25-26 are perspective views of alternate embodiments of absorbent articles.

### DETAILED DESCRIPTION OF THE INVENTION

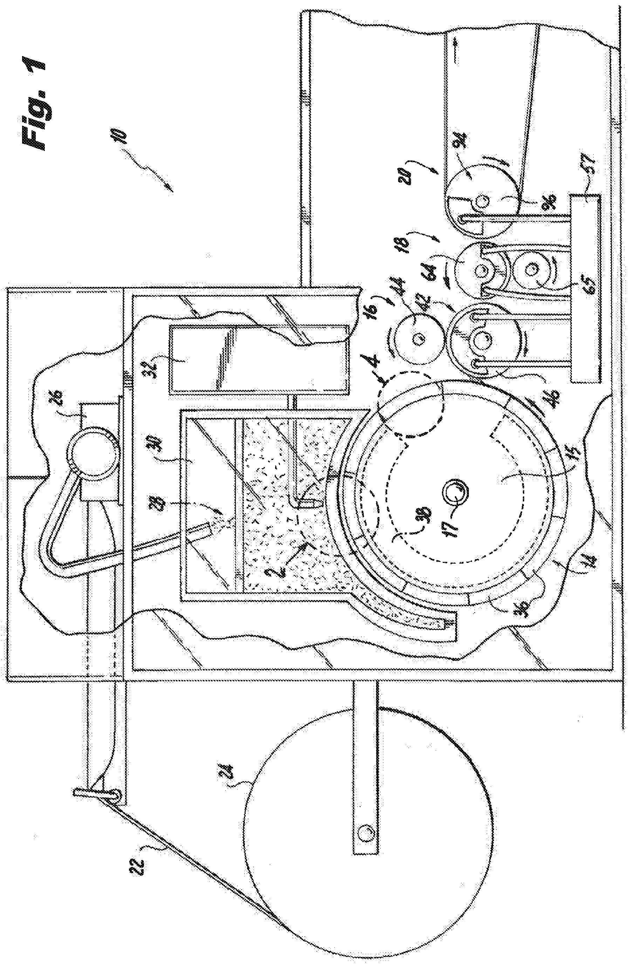

Referring to Figs. 1-9 and 11-20, there is illustrated a preferred apparatus 10 for making a formed fibrous article 12 according to the method of the present invention.

As shown in Fig. 1, the apparatus 10 according to the present invention generally includes a forming drum 14, a calendering station 16, a pin calendering station 18, and a transfer wheel 20. Certain details of the apparatus 10, such as electrical lines, have been omitted from the figures to simplify the same. However, these features and other basic elements of the apparatus will be clear to those of skill in the art.

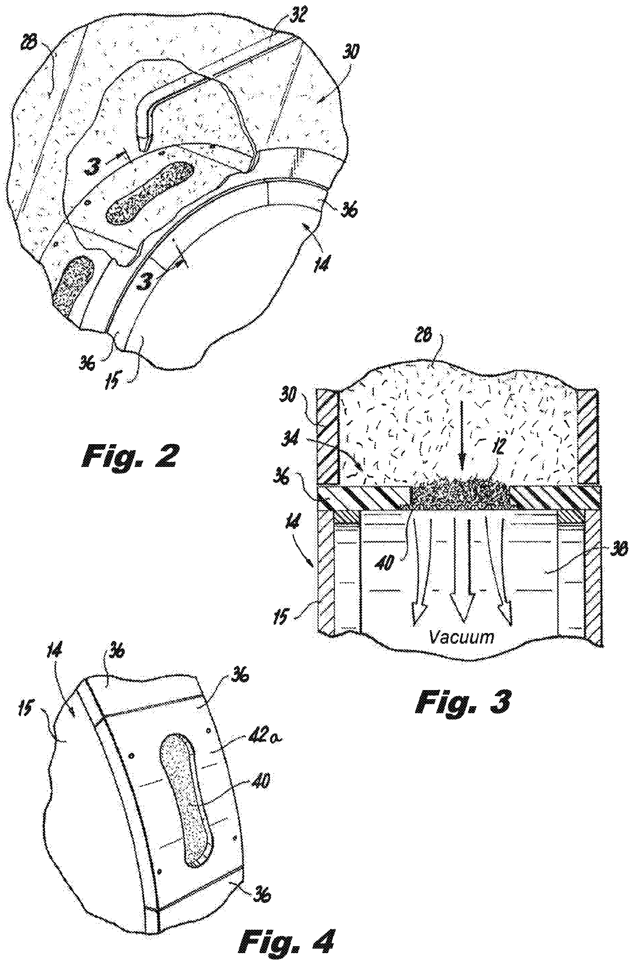

The formed fibrous article 12, which is depicted during various stages of the method according to the present invention in Figs. 3, 5-6, 8, 10, 12 and 18-22, preferably is formed from cellulosic fibers, and in a preferred embodiment of the invention, includes a mixture of cellulosic fibers and superabsorbent polymer. Cellulosic fibers that can be used in the formed fibrous article 12 are well known in the art and include wood pulp, cotton, flax and peat moss. Wood pulp is preferred. Both softwood and hardwood species are useful. Softwood pulps are preferred.

The fibrous article 12 may also contain any superabsorbent polymer (SAP), which are well known in the art. For the purposes of the present invention, the term "superabsorbent polymer" (or "SAP") refers to materials, which are capable of absorbing and retaining at least about 10 times their weight in body fluids under a 3.4 kPa (0.5 psi) pressure. The superabsorbent polymer particles may be inorganic or organic cross-linked hydrophilic polymers, such as polyvinyl alcohols, polyethylene oxides, cross-linked starches, guar gum, xanthan gum, and the like. The particles may be in the form of a powder, grains, granules, or fibers. Preferred superabsorbent polymer particles for use in the present invention are crosslinked polyacrylates, such as the product offered by Sumitomo Seika Chemicals Co., Ltd. of Osaka, Japan, under the designation of SA70N and products offered by Stockhausen Inc..

The pulp used to form the fibrous article 12 is preferably a bleached softwood pulp, produced by a Kraft process. As shown in Fig. 1, the pulp is provided by the manufacturer as a pulp board 22 in rolled form, the roll identified by the reference numeral 24. The pulp board 22 is conveyed from the roll 24 to a device 26 for grinding the pulp board 22 into fibrous pulp 28. The fibrous pulp 28 is released from the grinding device 26 into a chamber 30 for holding the fibrous pulp 28. The apparatus 10 may further optionally include a device 32 for introducing superabsorbent polymer into the chamber 30 to thereby form a fibrous pulp and superabsorbent mixture. Any conventional device suitable for this purpose, and known to those of skill in the art, may be used for introducing the superabsorbent into the chamber 30.

As best seen in Fig. 3, the chamber 30 has a partially open bottom portion 34 that communicates with the forming drum 14. As seen in Fig. 1, the forming drum 14 includes a hollow cylinder 15 that is structured and arranged to rotate about a fixed axis 17. Any conventional means to rotate the cylinder 15, well known to those of skill in the art, may be used to rotate the cylinder 15. As shown in Figs. 1-4, the cylinder 15 has a plurality of molds 36 mounted thereto. As the cylinder 15 rotates, each of the molds 36 are sequentially arranged in communication with the open portion 34 of the chamber 30 to thereby receive fibrous pulp 28 from the chamber 30. In Figure 1, the cylinder 15 rotates in a counterclockwise manner during operation of the apparatus 10.

As shown in Figs. 1 and 3, the forming drum 14 further includes a vacuum chamber 38 arranged within the interior of the cylinder 15. The vacuum chamber 38 is arranged in a fixed location relative to the rotating cylinder 15 and is operably coupled to a vacuum source (not shown). As best seen in Fig. 4, the mold 36 includes a porous screen 40 structure in the shape of the formed fibrous article 12 to be formed in the mold 36. As the mold 36 passes over the vacuum chamber 38 of the forming drum 14 the vacuum functions to draw the fibrous pulp 28 from the chamber 30 into the mold 36 by drawing air through the porous screen 40 of the mold 36.

As shown in detail in Fig. 4, the mold 36 includes a nonporous mounting plate portion 42a that surrounds the porous screen 40 portion of the mold 36. The mounting plate portion 42a of the mold 36 is mounted to the periphery 4 of the cylinder 15, thereby enabling each of the molds 36 to rotate with the rotating cylinder 15.

After the mold 36 is rotated under the partially open bottom portion 34 of the chamber 30, the mold 36 is further rotated by the rotating cylinder 15, and as described in further detail below, the fibrous article 12 is then transferred to the calendering station 16.

After formation in the mold 36, the formed fibrous article 12 preferably has a basis weight in the range of between about 200 gsm (g/m²) to about 400 gsm, a thickness in the range of about 5 mm to about 20 mm, and a density in the range of about 0.01 g/cc to about 0.03 g/cc.

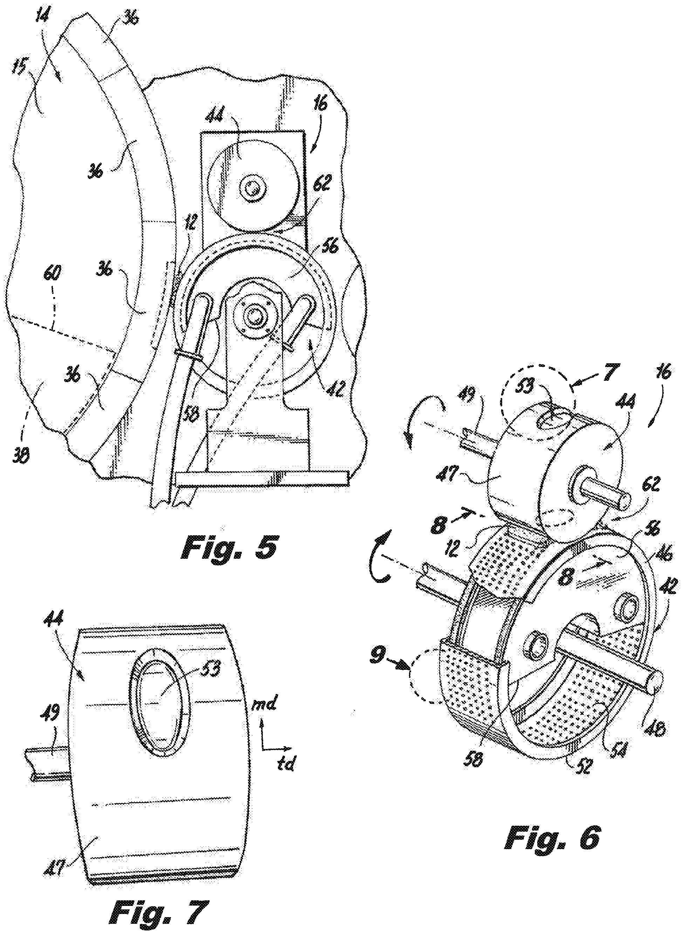

As shown in Figs. 1 and 5-9 the calendering station 16 includes a vacuum roll 42 and an opposed calender roll 44. As best seen in Fig. 6, the vacuum roll 42 is formed from a rotatable cylinder 46 that is rotatable about a fixed axis 48. The calender roll 44 is rotateable about a fixed axis 49. Any conventional means to rotate the cylinder 46, and calender roll 44, well known to those of skill in the art, may be used to rotate cylinder 46 and calender roll 44. The cylinder 46 rotates in a clockwise manner during operation of the apparatus 10, and the calender roll 44 rotates in a counterclockwise manner, as show in Fig. 1. As best seen in Figs. 6, 8 and 9, the cylinder 46 includes a plurality of holes 50 that extend from the outer surface 52 of the cylinder 46 to the inner surface 54 of the cylinder 46.

As shown in detail in Fig. 7, the surface 47 of the calender roll 44 includes a recess 53. The recess 53 may take any number of different shapes including generally oval, elliptical, circular or the like. In one preferred embodiment of the invention, as shown in Fig. 7, the recess generally has an oval shape. Preferably, the recess 53 extends over a surface area in range of about 500 mm² to about 5000 mm². The recess 53 preferably has a depth in the range of about 2 mm to about 25 mm as measured from the surface 47 of the roll 44 located outside the recess 53. The recess 53 is preferably arranged such that it is longer in the machine direction (md) than in the transverse direction (td). Preferably, the recess 53 has a maximum length, as measured in the machine direction, in the range of about 20 mm to about 120 mm and a maximum width, as measured in the transverse direction, in the range of about 5 mm to about 60 mm.

As shown in Figs. 5, 6 and 8, the vacuum roll 42 further includes a vacuum chamber 56 arranged within the interior of the cylinder 46. The vacuum chamber 56 is arranged in a fixed location relative to the rotating cylinder 46 and is operably coupled to a vacuum source 57 (Fig. 1). The vacuum chamber 56 is arranged in flow communication with the plurality of holes 50 that extend through the cylinder 46 and thereby draws air through said holes 50.

As shown in Fig. 5, the vacuum chamber 56 is arranged such that its leading edge 58 is substantially aligned with a trailing edge 60 of the vacuum chamber 38 located within cylinder 15 of the forming drum 14. This arrangement of the vacuum chamber 56 relative to location of the vacuum chamber 38 effectuates a transfer of the formed fibrous article 12 from within the mold 36 on the forming drum 14 to the vacuum roll 42.

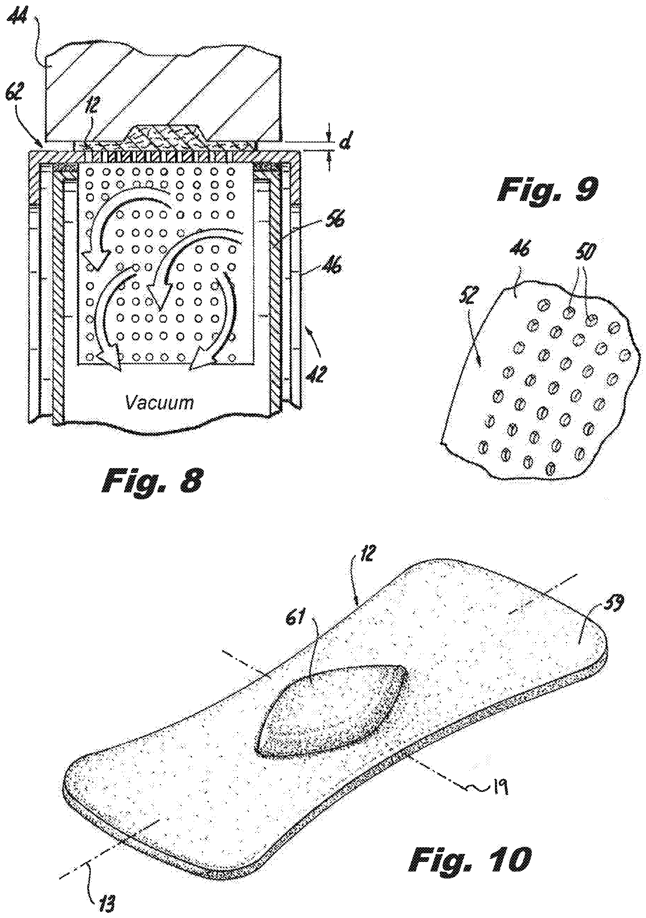

Once the formed fibrous article 12 has been transferred to the cylinder 46 of the vacuum roll 42 the cylinder 46 rotates the fibrous 12 article until the article 12 passes through the nip 62 formed between the vacuum roll 42 and the calender roll 44. The nip 62 preferably uniformly compresses the fibrous article 12 outside of the area defined by recess 53. The compression of the fibrous article 12 results in a reduction in thickness of the article 12 and a corresponding increase in density in that portion of the fibrous article 12 located outside of the area of the recess 53. In a preferred embodiment of the invention the nip 62 has a distance "d" (i.e. the distance between the surfaces of the opposed rolls) of about 0.9 mm. The distance "d" is indentified by the reference symbol "d" in Fig. 8.

As shown in Fig. 10, after passing through the nip 62 the fibrous article 12 generally includes two areas 59 and 61. The first area 59, corresponds to that portion of the article 12 that has been compressed by the rolls 42 and 44 outside of the area of the recess 53. Area 59 preferably extends over a surface area of between about 7000 mm² and 14000 mm², has a thickness in the range of about 4 mm to about 12 mm, and a density in the range of about 0.02 g/cc to about 0.1 g/cc. Area 61 of the article 12 is that portion of the article 12 corresponding in location to the recess 53. Area 61 preferably extends over a surface area of between about 1000 mm² and 7000 mm², has a thickness in the range of about 10 mm to about 20 mm, and a density in the range of about 0.01 g/cc to about 0.04 g/cc.

It is noted that the fibrous article 12, after passing through the nip 62, has a three dimensional profile. Specifically, area 59 of the fibrous article defines a substantially planar portion of the fibrous article 12 and area 61 extends upwardly from the substantially planar portion thereby defining an upwardly extending hump or raised portion. In one preferred embodiment of the invention, as shown in Fig. 10, area 61 is symmetrically arranged with respect to longitudinally extending central axis 13 of the fibrous article and the transversely extending central axis 19 of the fibrous article 12

It is noted that the fibrous article 12 has a three dimensional profile yet has a constant basis weight throughout its entire structure. Specifically, planar portion 59 and the raised portion 61 are both formed from a common material composition having a constant basis weight, yet differ in thickness and density. It is also noted that the entire fibrous article 12 is formed from a single material layer.

After the article 12 passes through the nip 62 the article 12 is further rotated in a clockwise direction by the cylinder 46 of the vacuum roll 42, and as will be described in further detail below, transferred to the pin calendering station 18.

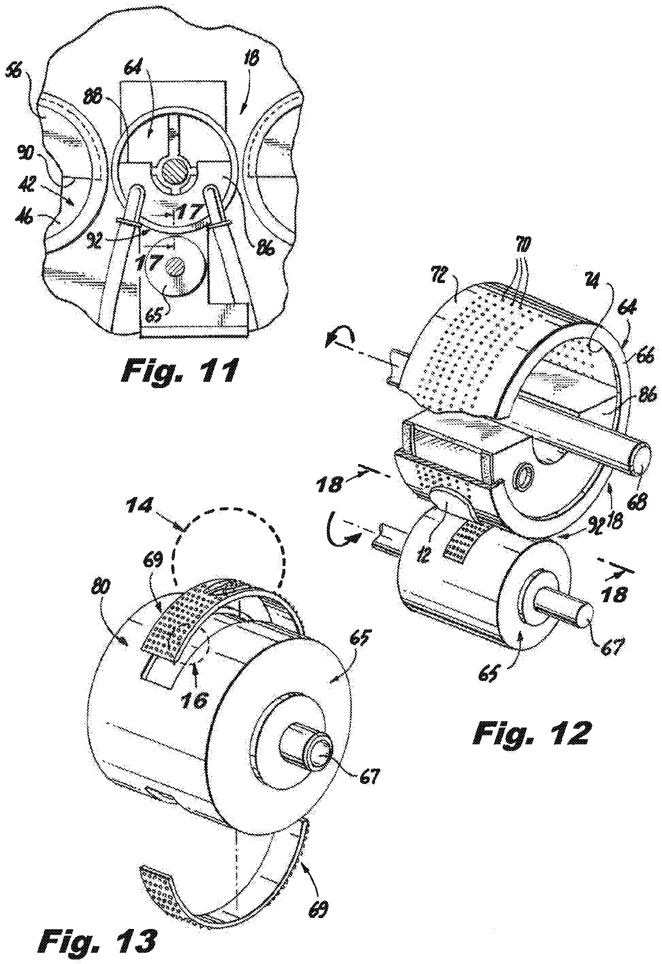

As shown in Figs. 1 and 11-18 the pin calendering station 18 includes a vacuum roll 64 and an opposed pin calender roll 65. As best seen in Fig. 12, the vacuum roll 64 is formed from a rotatable cylinder 66 that is rotatable about a fixed axis 68. Any conventional means to rotate the cylinder 66, well known to those of skill in the art, may be used to rotate the cylinder 66. The cylinder 66 rotates in a counterclockwise direction during operation of the apparatus 10. As shown in Fig. 12, the cylinder 66 includes a plurality of holes 70 that extend from the outer surface 72 of the cylinder 66 to the inner surface 74 of the cylinder 66. In a preferred embodiment of the invention each of the plurality of holes 70 has a diameter of about 1.5 mm and is spaced from an adjacent hole by a distance of about 4 mm (center to center).

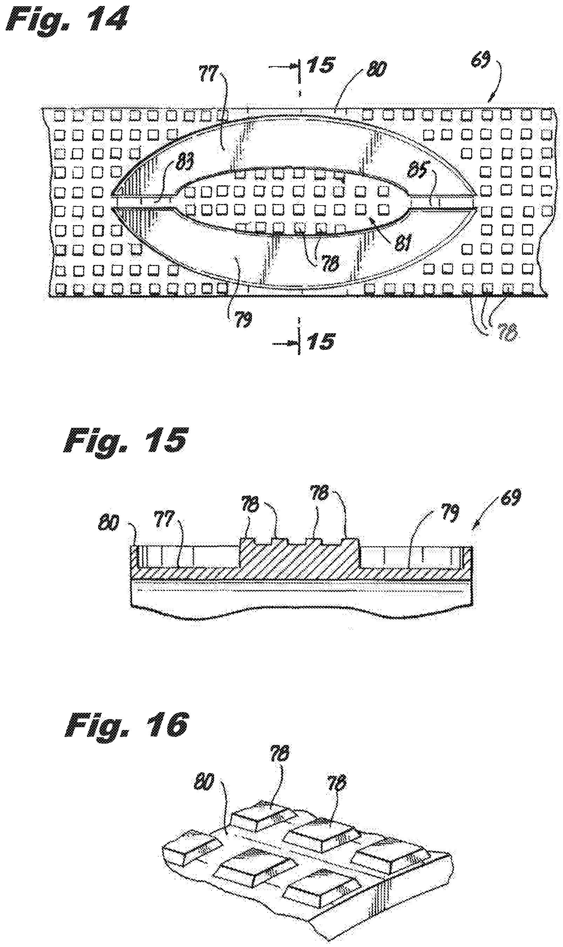

As shown in Figs. 12 and 13 the pin calender roll 65 is structured and arranged to rotate about a fixed axis 67. Any conventional means to rotate the pin calender roll, well known to those of skill in the art, may be used to rotate the roll 65. As shown in Figs. 13 and 14 the pin calender roll 65 has a roll surface 80 including a first area 69 having plurality of individual pins 78 that extend outwardly from a surface 80 of the roll 65. As shown in Fig. 13, the first area 69 extends down a central portion of the roll surface 80. In a preferred embodiment of the invention, the first area 69 is structured and arranged to emboss a central region of the fibrous article 12. Alternatively, the first area 69 may be arranged to emboss substantially the entire fibrous article 12.

As best seen in Fig. 14, the surface 80 of the roll 65 is further provided with a first recess 77 and a second recess 79. Each of the first recess 77 and second recess 79 are structured and arranged to correspond in location to area 61 of the fibrous article 12 when the article 12 passes through the nip 92 defined between the vacuum roll 64 and the pin calender roll 65. Preferably each recess 77 and 79 extends over a surface area of between about 260 mm² and 1100 mm² and has a depth of between about 2 mm and 25 mm.

As shown in Fig. 14, in a preferred embodiment of the invention, each recess 77 and 79 is generally arcuate in shape and generally extends in a machine direction. Each recess 77 and 79 is preferably positioned on the roll 65 so that it is symmetrically arranged with respect to the other recess about the longitudinally extending central axis 13 of the fibrous article and the transversely extending central axis 19 of the fibrous article 12, as the article 12 passes through the through the nip 92 defined between the vacuum roll 64 and the pin calender roll 65.

Recess 77 is separated from recess 79 by a land area 81 that includes a plurality of pins 78. Preferably the land area 81 has a surface area of between about 250 mm² and 1000 mm². The land area 81 is connected to the first area 67 by a first smooth roll surface segment 83 and second smooth roll surface segment 85, each of the segments 83 and 85 being preferably free of pins 78.

In preferred embodiments of the invention each of the pins 78 are spaced from an adjacent pin by a distance of about 4 mm (center to center), have height of about 1.5 mm and have an effective contact area of from about 0.8 mm² to about 1.2 mm².

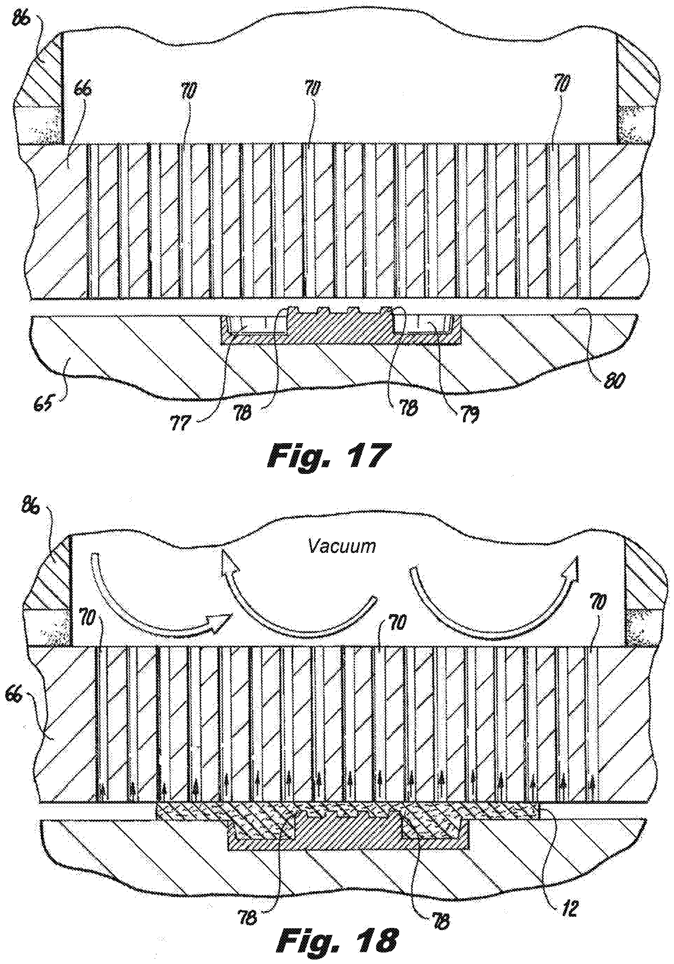

As shown in Figs. 17 and 18, each of the pins 78 are arranged such that they do not overlap with any of the plurality of holes 70 in the cylinder 66 of the vacuum roll 64. This arrangement of the pins 78 relative to the holes 70 insures that no pulp is forced into any of the plurality of holes 70, thereby improving the efficiency of pulp use and the efficiency of the process as a whole.

As shown in Fig. 11-12 and 17-18, the vacuum roll 64 further includes a vacuum chamber 86 arranged within the interior of the cylinder 66. The vacuum chamber 86 is arranged in a fixed location relative to the rotating cylinder 66 and is operably coupled to the vacuum source 57 (Fig. 1). The vacuum chamber 86 is arranged in flow communication with the plurality of holes 70 that extend through the cylinder 66 and thereby draws air through said holes 70.

As shown in Fig. 11, the vacuum chamber 86 is arranged such that its leading edge 88 is substantially aligned with a trailing edge 90 of the vacuum chamber 56 located within the cylinder 46 of the vacuum roll 42. This arrangement of the vacuum chamber 86 relative to the location of the vacuum chamber 56 effectuates a transfer of the formed fibrous article 12 from the vacuum roll 42 to the vacuum roll 64.

Once the formed fibrous article 12 has been transferred to the cylinder 66 of the vacuum roll 64, the cylinder 66 rotates the formed fibrous article 12 until the article 12 passes through the nip 92 formed by the vacuum roll 64 and the pin calender roll 65.

In a preferred embodiment of the invention the nip 92 has a distance (i.e. the distance between the surfaces of the opposed rolls) of about 0.8 mm. Also in a preferred embodiment of the invention the pin calender roll 65 is heated to a temperature of between about 80° C (176° F) to about 100° C (212 °F) by means of any suitable conventional heating means. It has been found that heating the pin calender roll 65 in this manner helps prevent the formed fibrous article 12 from adhering to the surface of the pin calender roll 65.

After the article 12 passes through the nip 92 the article 12 is further rotated in a counter clockwise direction by the cylinder 66 of the vacuum roll 64 as shown and, as will be described in further detail below, transferred to the transfer wheel 20.

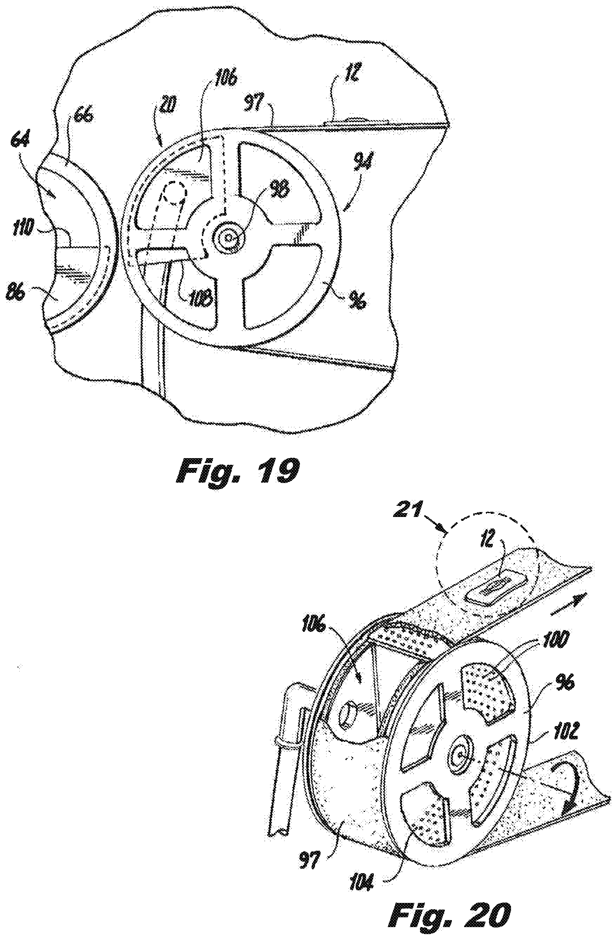

As shown in Fig. 19 the transfer wheel 20 comprises a vacuum roll 94 that is formed from a rotatable cylinder 96 that is rotatable about a fixed axis 98. Any conventional means to rotate the cylinder 96, well known to those of skill in the art, may be used to rotate the cylinder 96. The cylinder 96 rotates in a counterclockwise manner during operation of the apparatus 10, as show in Fig. 1. As shown in Fig. 20, the cylinder 96 includes a plurality of holes 100 that extend from the outer surface 102 of the cylinder 96 to the inner surface 104 of the cylinder 96.

As shown in Figs. 19 and 20, the vacuum roll 94 further includes a vacuum chamber 106 arranged within the interior of the cylinder 96. The vacuum chamber 106 is arranged in a fixed location relative to the rotating cylinder 96 and is operably coupled to the vacuum source 57 (Fig. 1). The vacuum chamber 106 is arranged in flow communication with the plurality of holes 100 that extend through the cylinder 96 and thereby draw air through said holes 100.

The transfer wheel 20 further includes a porous conveyor belt 97 that extends around the cylinder 96 and travels with the cylinder 96, i.e. in a clockwise direction shown in Fig. 20.

As shown in Fig. 19, the vacuum chamber 106 is arranged such that its leading edge 108 is substantially aligned with a trailing edge 110 of the vacuum chamber 86 located within the cylinder 66 of the vacuum roll 64. This arrangement of the vacuum chamber 106 relative to the location of the vacuum chamber 86 effectuates a transfer of the formed fibrous article 12 from the vacuum roll 64 to the vacuum roll 94. Specifically, formed fibrous article 12 is transferred to the conveyor belt 97 and held in place by the vacuum chamber 106 that functions to draw air through the porous conveyer belt 97 via the holes 100 in the cylinder 96.

Once the fibrous article 12 is rotated past vacuum chamber 106, the porous conveyer belt 97 functions to further convey the formed fibrous article 12 in a machine direction. The formed fibrous article 12 may be conveyed in a machine direction for incorporation into a final product structure such as a sanitary napkin, panty liner, incontinence article, diaper or the like.

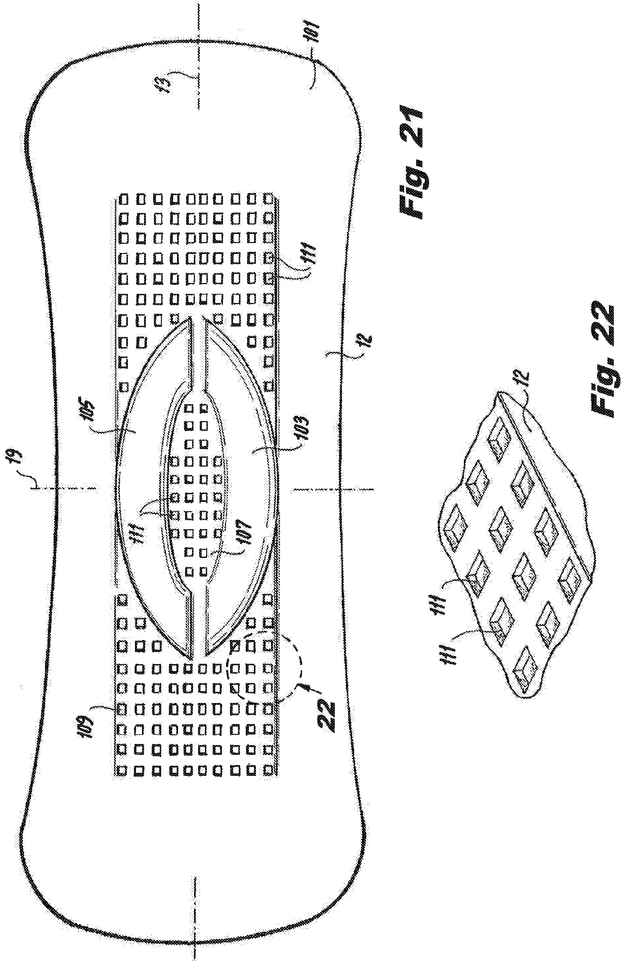

Referring to Figs. 21 and 22, the completed fibrous article 12 generally includes an area 101 that has not been pin embossed, a first arcuate raised area 103, a second arcuate raised area 105, a pin embossed region 107 located between the raised area 103 and raised area 105, and a centrally extending pin embossed area 109 extending along the longitudinally extending central axis 13 of the article 12. Each of the pin embossed regions 107 and 109 include a plurality of depressions 111 corresponding in location to the pins 78. Areas 101 and 109 cooperate to define a substantially planar portion of the fibrous article 12 and raised areas 103 and 105 extend upwardly relative to the planar portion of the fibrous article 12.

After passing through the nip 92 area 101 preferably has a thickness in the range of about 0.8 mm to about 3.5 mm, a density in the range of about 0.06 g/cc to about 0.5 g/cc, and extends over a surface area of from about 6400 mm² and 9400 mm².

After passing through the nip 92 each of area 103 and 105 preferably has a thickness in the range of about 2 mm to about 10 mm and a density in the range of about 0.01 g/cc to about 0.1 g/cc. Each area 103 and 105 preferably extends over a surface area of from about 260 mm² and 1100 mm².

After passing through the nip 92 area 107 preferably has a thickness in the range of about 0.2 mm to about 1 mm, a density in the range of about 0.1 g/cc to about 0.9 g/cc in those areas 111 embossed by the pins 78 and a thickness in the range of about 0.8 mm to about 3.5 mm and a density in the range of about 0.06 g/cc to about 0.5 g/cc in those areas outside of areas 111. Area 107 preferably extends over a surface area of from about 250 mm² and 1000 mm².

After passing through the nip 92 area 109 preferably has a thickness in the range of about 0.2 mm to about 1.0 mm and a density in the range of about 0.1 g/cc to about 0.9 g/cc in those areas 111 embossed by the pins 78 and a thickness in the range of about 0.8 mm to about 3.5 mm and a density in the range of about 0.06 g/cc to about 0.5 g/cc in those areas outside of areas 111. Area 109 preferably extends over a surface area of from about 2400 mm² and 7600 mm².

It is noted that although the different areas of the fibrous article 12 differ in density and thickness the fibrous article 12 has a uniform basis weight throughout its entire structure. It is further noted that although fibrous article 12 possesses at least one raised area, i.e. the article 12 possesses a three dimensional profile, the article 12 has a uniform basis weight. Preferred examples also present the above characteristics while being formed from a uniform material composition. In addition, in preferred examples, the fibrous article 12 is formed from a single material layer.

It is noted that the formed fibrous article 12 is formed and pin-calendered without the use of any "carrier layer". In addition, is noted that the fibrous article is transferred from the forming drum 14 to the calendering station 16 then to the pin calendering station 18 without the use of any "carrier layer". "Carrier layer" as used herein means any material layer used to support the fibrous article, such as a conveyer belt or an adjacent material layer such a rolled nonwoven layer or the like.

In connection with the various vacuum chambers disclosed herein any suitable vacuum source may be employed. In a preferred embodiment of the invention the vacuum source is an air blower having an air flow of about 2,200 cubic meters per hour.

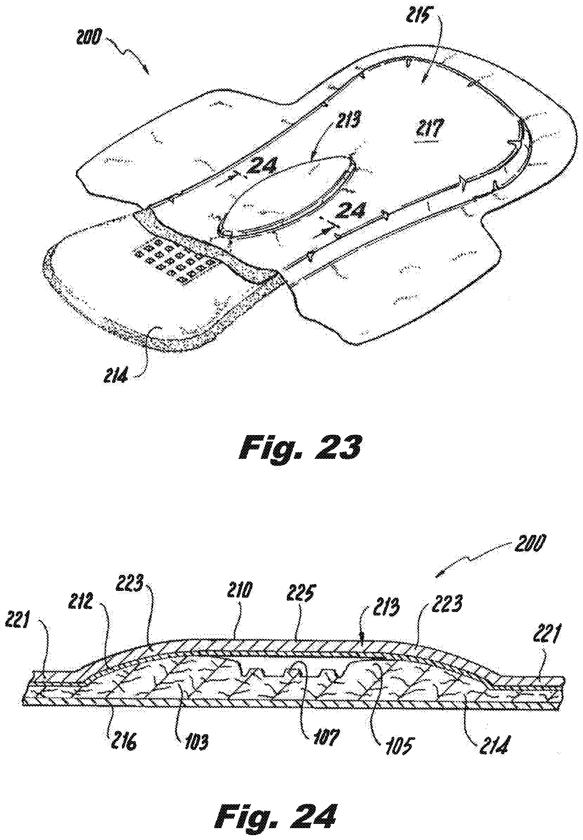

Reference is made to Figs. 23-24 which depict a disposable absorbent article 200. Although disposable absorbent articles will be described herein with reference to a sanitary napkin 200, other disposable absorbent articles are panty liners, adult incontinence articles, and diapers. As shown in Fig. 24, the sanitary napkin 200 includes a liquid permeable cover layer 210, an optional transfer layer 212, an absorbent core 214 and a liquid impermeable barrier layer 216. The absorbent core 214 layer is formed from a formed fibrous article 12 of the type described above.

As shown in Fig. 23, the absorbent article 200 includes a raised area 213 that extends upwardly from the remaining body facing planar portion 215 of the napkin 200. Specifically the raised area 213 extends upwardly from a top surface 217 of the planar portion 215. Preferably, the raised area 213 extends upwardly a distance of about 2 mm to about 10 mm as measured from the top surface 217 of the planar portion 215 and extends over a surface area of between about 1000 mm² and 7000 mm². Preferably, the planar portion 215 extends over an area of between about 7000 mm² and 14000 mm².

### Main Body-Cover Layer

The cover layer 210 may be a relatively low density, bulky, high-loft non-woven web material. The cover layer 210 may be composed of only one type of fiber, such as polyester or polypropylene or it may include a mixture of more than one fiber. The cover may be composed ofbi-component or conjugate fibers having a low melting point component and a high melting point component. The fibers may be selected from a variety of natural and synthetic materials such as nylon, polyester, rayon (in combination with other fibers), cotton, acrylic fiber and the like and combinations thereof. Preferably, the cover layer 210 has a basis weight in the range of about 10 gsm to about 75 gsm.

Bi-component fibers may be made up of a polyester layer and a polyethylene sheath. The use of appropriate bi-component materials results in a fusible non-woven fabric. Examples of such fusible fabrics are described in U.S. Pat. No. 4,555,430 issued Nov. 26, 1985 to Chicopee. Using a fusible fabric increases the ease with which the cover layer may be mounted to the absorbent layer(s) of the article and/or to the barrier layer 216.

The cover layer 210 preferably has a relatively high degree of wettability, although the individual fibers comprising the cover may not be particularly hydrophilic. The cover material should also contain a great number of relatively large pores. This is because the cover layer 210 is intended to take-up body fluid rapidly and transports it away from the body and the point of deposition. Therefore, the cover layer contributes little to the time taken for the napkin 200 to absorb a given quantity of liquid (penetration time).

Advantageously, the fibers that make up the cover layer 210 should not lose their physical properties when they are wetted, in other words they should not collapse or lose their resiliency when subjected to water or body fluid. The cover layer 210 may be treated to allow fluid to pass through it readily. The cover layer 210 also functions to transfer the fluid quickly to the underlying layers of the absorbent article. Thus, the cover layer 210 is advantageously wettable, hydrophilic and porous. When composed of synthetic hydrophobic fibers such as polyester or bi-component fibers, the cover layer 210 may be treated with a surfactant to impart the desired degree of wettability.

The cover layer 210 may be made from a 27 gsm hot through air (HTA) bonded nonwoven material constructed from 100% bico fibers (PE/PET), commercially available from Shalag Industries A.C.S. Ltd., Kibbutz Shamir, Upper Galilee, Israel, under the commercial code STA4ETW27.

Alternatively, the cover layer 210 can also be made of a polymer film having large pores. Because of such high porosity, the film accomplishes the function of quickly transferring body fluid to the underlying layers of the absorbent article. A suitable cover material of this type is commercially found on the Stayfree Dry Max Ultrathin product distributed by McNeil-PPC, Inc.

The cover layer 210 may be embossed to the underlying absorbent layers in order to aid in promoting hydrophilicity by fusing the cover to the adjacent underlying layer. Such fusion may be effected locally, at a plurality of sites or over the entire contact surface of cover layer 210. Alternatively, the cover layer 210 may be attached to the other layers of the article by other means such as by adhesion.

### Main Body -- Transfer Layer

Adjacent to the cover layer 210 on its inner side and bonded to the cover layer 210 is the optional transfer layer 212. The transfer layer 212 provides means for receiving body fluid from the cover layer 210 and holding it until the underlying absorbent core 214 has an opportunity to absorb the fluid, and therefore acts as a fluid transfer or acquisition layer. The transfer layer 212 is, preferably, more dense than and has a larger proportion of smaller pores than the cover layer 210. These attributes allow the transfer layer 212 to contain body fluid and hold it away from the outer side of the cover layer 210, thereby preventing the fluid from rewetting the cover layer 210 and its surface. However, the transfer layer is, preferably, not so dense as to prevent the passage of the fluid through the layer 212 into the underlying absorbent core 214.

The transfer layer 212 may be composed of fibrous materials, such as wood pulp, polyester, rayon, flexible foam, or the like, or combinations thereof. The transfer layer 212 may also comprise thermoplastic fibers for the purpose of stabilizing the layer and maintaining its structural integrity. The transfer layer 212 may be treated with surfactant on one or both sides in order to increase its wettability, although generally the transfer layer 212 is relatively hydrophilic and may not require treatment. The transfer layer 212 is preferably bonded or adhered on both sides to the adjacent layers, i.e. the cover layer 210 and the underlying absorbent core 214.

Examples of suitable materials for the transfer layer 212 are through air bonded pulp sold by Buckeye Technologies of Memphis, Tenn., under the designation VIZORB 3008, which has a basis weight of 110 gsm, VIZORB 3042, which has a basis weight of 100 gsm, and VIZORB 3010, which has a basis weight of 90 gsm.

### Main Body -- Absorbent Core

Referring to Fig. 18, and as discussed above, the absorbent article 200 includes an absorbent core 214. The absorbent core 214 consists of a formed fibrous article 12 of the type described herein above.

The absorbent core 214 may be a blend or mixture of cellulosic fibers and superabsorbent disposed therein. Cellulosic fibers that can be used in the absorbent core 214 are well known in the art and include wood pulp, cotton, flax and peat moss. Wood pulp is preferred.

The absorbent core 214 can contain any superabsorbent polymer (SAP), which are well known in the art. For the purposes of the present invention, the term "superabsorbent polymer" (or "SAP") refers to materials, which are capable of absorbing and retaining at least about 10 times their weight in body fluids under a 3.4 kPa (0.5 psi) pressure. The superabsorbent polymer particles may be inorganic or organic crosslinked hydrophilic polymers, such as polyvinyl alcohols, polyethylene oxides, crosslinked starches, guar gum, xanthan gum, and the like. The particles may be in the form of a powder, grains, granules, or fibers. Preferred superabsorbent polymer particles for use in the present invention are crosslinked polyacrylates, such as the product offered by Sumitomo Seika Chemicals Co., Ltd. of Osaka, Japan, under the designation of SA70N and products offered by Stockhausen Inc..

The absorbent core 214 preferably has a total basis weight in the range of about 200 gsm to about 400 gsm. In preferred examples the absorbent core 214 includes about 50%-100% pulp by weight and about 0% to about 50% superabsorbent by weight.

As described above in the description of the method of making the fibrous article 12 set forth above, and in reference to Figs. 21 and 22, the absorbent core 214 generally includes an area 101 that has not been pin embossed, a first arcuate raised area 103, a second arcuate raised area 105, a pin embossed region 107 located between the raised area 103 and raised area 105, and a centrally extending pin embossed area 109. Referring to Fig. 24, it is noted that the region 107 located between the raised areas 103 and 105 is recessed related to the raised areas. That is, raised areas 103 and 105 have a greater thickness than region 107.

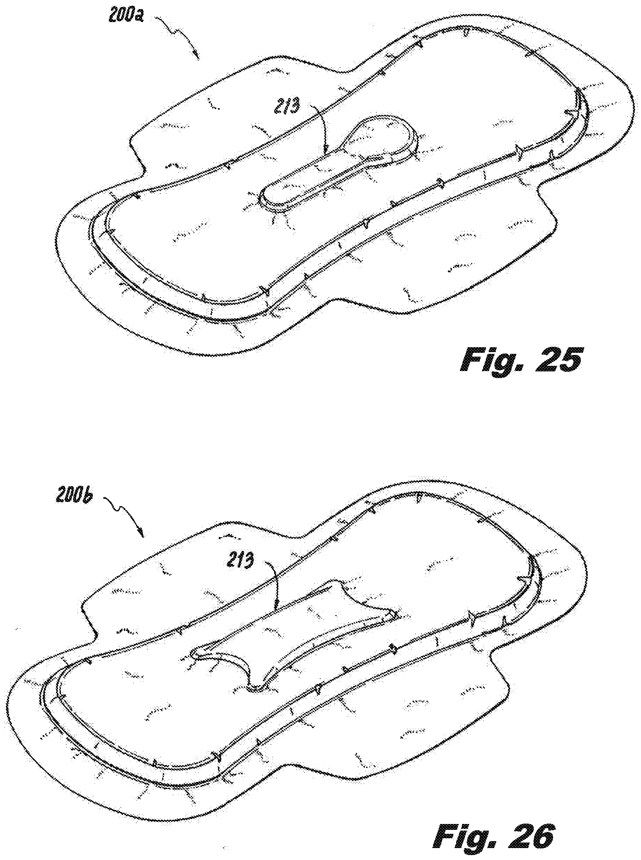

As shown in Fig. 24, the first arcuate raised area 103 and the second arcuate raised area 105 of the absorbent core 214 correspond in location, and help define, the raised area 213 of the napkin 200. However, it is noted that the final shape of the raised area 213 of the napkin 200 is provided by a conventional embossing step (not shown in the figures) and thus the shape of the raised area 213 is not dictated solely by the shape of the first arcuate raised area 103 and the second arcuate raised area 105. The raised area 213 of the napkin 200 may be formed to have any number of different shapes. For example, two alternate embodiments of the napkin 200a and 200b are depicted in Figs. 25 and 26. As shown, napkins 200a and 200b include raised areas 213 having different shapes than the napkin 200 shown in Figs. 23 and 24. Other shapes are also possible. In addition, although the napkin 200 is depicted as having only a single raised area 213 is possible that the napkin could be provided with a plurality of such raised areas 213.

All of the articles shown in Figs. 23-26 use absorbent cores having raised areas 103 and 105 as shown in Fig. 21, and the final shape of the raised area 213 has been modified merely by using a correspondingly shaped conventional embossing roll to emboss the napkin 200 after the various layers of the napkin 200 have been adhered to one another.

Referring to Fig. 24, it is noted that the cover layer extends over the first arcuate raised area 103, the second arcuate raised area 105, as well as the region 107 located between the raised area 103 and raised area 105. In this manner, the cover layer 210 generally includes a first portion 221 which is located in body facing planar portion 215 of the napkin 200 and is arranged in abutting surface to surface contact with the transfer layer 212 (or the absorbent core 214 if the transfer layer 212 is omitted), a pair of second regions 223 that are arranged in corresponding location to the arcuate raised areas 103 and 105 of the absorbent core 214, and a third region 225 that is located between the arcuate raised areas 103 and 105 and is arranged in spaced relationship to the absorbent core 214.

In one specific example, the absorbent core 214 consists of a 305 gsm fluff pulp and superabsorbent mixture, the mixture including about 89 % fluff pulp by weight, commercially available as Golden Isles Fluff Pulp 420#HD 7% Moisture, from GP Cellulose, Brunswick, Georgia, USA, and 11 % superabsorbent polymer by weight, commercially available as Aqua Keep SA70N from Sumitomo Seika Chemicals Co., Ltd., Osaka, Japan.

### Main Body-Barrier Layer

Underlying the absorbent core 214 is a barrier layer 216 comprising liquid-impervious film material so as to prevent liquid that is entrapped in the absorbent core 214 from egressing the sanitary napkin 200 and staining the wearer's undergarment. The barrier layer 216 is preferably made of polymeric film, although it may be made of liquid impervious, air-permeable material such as repellent-treated non-woven or micropore films or foams.

The barrier layer may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet. The cover layer 210 and the barrier layer 216 are joined along their marginal portions so as to form an enclosure or flange seal that maintains the absorbent core 214 captive. The joint may be made by means of adhesives, heat-bonding, ultrasonic bonding, radio frequency sealing, mechanical crimping, and the like and combinations thereof.

In one specific example, the barrier layer consists of a liquid impermeable 24 gsm polyethylene film commercially available from Clopay do Brasil, Sau Paulo, SP, Brazil.

Positioning adhesive may be applied to a garment facing side of the barrier layer 216 for securing the napkin 200 to the garment during use. The positioning adhesive may be covered with removable release paper so that the positioning adhesive is covered by the removable release paper prior to use.

Absorbent articles may or may not include wings, flaps or tabs for securing the absorbent article to an undergarment. Wings, also called, among other things, flaps or tabs, and their use in sanitary protection articles is described in U.S. Patent. No. 4,687,478 to Van Tilburg; U.S. Patent No. 4,589,876 also to Van Tilburg, U.S. Patent No. 4,900,320 to McCoy, and U.S. Patent No. 4,608,047 to Mattingly.

The sanitary napkin 200 may be applied to the crotch by placing the garment-facing surface against the inside surface of the crotch of the garment. Various methods of attaching absorbent articles may be used. For example, chemical means, e.g., adhesive, and mechanical attachment means, e.g., clips, laces, ties, and interlocking devices, e.g., snaps, buttons, VELCRO (Velcro USA, Inc., Manchester, NH), zipper, and the like are examples of the various options available to the artisan.

Adhesive may include pressure sensitive adhesive that is applied as strips, swirls, or waves, and the like. As used herein, the term pressure-sensitive adhesive refers to any releasable adhesive or releasable tenacious means. Suitable adhesive compositions, include, for example, water-based pressure-sensitive adhesives such as acrylate adhesives. Alternatively, the adhesive composition may include adhesives based on the following: emulsion or solvent-borne adhesives of natural or synthetic polyisoprene, styrenebutadiene, or polyacrylate, vinyl acetate copolymer or combinations thereof; hot melt adhesives based on suitable block copoylmers - suitable block copolymers for use in the invention include linear or radial co-polymer structures having the formula (A-B)x wherein block A is a polyvinylarene block, block B is a poly(monoalkenyl) block, x denotes the number of polymeric arms, and wherein x is an integer greater than or equal to one. Suitable block A polyvinylarenes include, but are not limited to Polystyrene, Polyalpha-methylstyrene, Polyvinyltoluene, and combinations thereof. Suitable Block B poly(monoalkenyl) blocks include, but are not limited to conjugated diene elastomers such as for example polybutadiene or polyisoprene or hydrogenated elastomers such as ethylene butylene or ethylene propylene or polyisobutylene, or combinations thereof. Commercial examples of these types of block copolymers include KratonTM elastomers from Shell Chemical Company, VectorTM elastomers from Dexco, SolpreneTM from Enichem Elastomers and StereonTM from Firestone Tire & Rubber Co.; hot melt adhesive based on olefin polymers and copolymers where in the olefin polymer is a terpolymer of ethylene and a co-monomers, such as vinyl acetate, acrylic acid, methacrylic acid, ethyl acrylate, methyl acrylate, n-butyl acrylate vinyl silane or maleic anhydride. Commercial examples of these types of polymers include Ateva(polymers from AT plastics), Nucrel(polymers from DuPont), Escor (from Exxon Chemical).

Any or all of the cover layer 210, transfer layer 212, absorbent core 214, barrier layer 216, and adhesive layers may be colored. Such coloring includes, but is not limited to, white, black, red, yellow, blue, orange, green, violet, and mixtures thereof. Color may be imparted according to the present invention through dying, pigmentation, and printing. Colorants used according the present invention include dyes and inorganic and organic pigments. The dyes include, but are not limited to, anthraquinone dyes (Solvent Red 111, Disperse Violet 1, Solvent Blue 56, and Solvent Green 3), Xanthene dyes (Solvent Green 4, Acid Red 52, Basic Red 1, and Solvent Orange 63), azine dyes (Jet black), and the like. Inorganic pigments include, but are not limited to, titanium dioxide (white), carbon black (black), iron oxides (red, yellow, and brown), chromium oxide (green), ferric ammonium ferrocyanide (blue), and the like.

Organic pigments include, but are not limited to diarylide yellow AAOA (Pigment Yellow 12), diarylide yellow AAOT (Pigment Yellow 14), phthalocyanine blue (Pigment Blue 15), lithol red (Pigment Red 49:1), Red Lake C (Pigment Red), and the like.

The sanitary napkin 200 may include other known materials, layers, and additives, such as, foam, net-like materials, perfumes, medicaments or pharmaceutical agents, moisturizers, odor control agents, and the like. The sanitary napkin 200 can optionally be embossed with decorative designs.

The sanitary napkin 200 may be packaged as unwrapped absorbent articles within a carton, box or bag. The consumer withdraws the ready-to-use article as needed. The sanitary napkin 200 may also be individually packaged (each absorbent article encased within an overwrap).

Also contemplated are asymmetrical and symmetrical absorbent articles having parallel longitudinal edges, dog bone- or peanut-shaped, as well as articles having a tapered construction for use with thong-style undergarments.

From the foregoing description, one skilled in the art can ascertain the essential characteristics of this invention, and without departing from the scope thereof, can make various changes and modifications. Embodiments set forth by way of illustration are not intended as limitations on the variations possible in practicing the present invention.

### EXAMPLES

Specific inventive examples of the present invention, and comparative examples, are described below.

### Inventive Example #1

An example of a sanitary napkin was constructed as follows. The body facing cover layer was constructed from a 27 gsm hot through air (HTA) bonded nonwoven material constructed from 100% bico fibers (PE/PET), commercially available from Shalag Industries A.C.S. Ltd., Kibbutz Shamir, Upper Galilee, Israel, under the commercial code STA4ETW27.

A 305 gsm formed fibrous absorbent core was arranged below the cover layer and was formed by the process described herein above with reference to Figs. 1-21.
The absorbent core had a composition of 89% by weight of pulp and 11% by weight of superabsorbent polymer. The pulp was Golden Isles Fluff Pulp 420#HD 7% Moisture, commercially available from GP Cellulose, Brunswick, Georgia, USA. The superabsorbent polymer was Aqua Keep SA70N commercially available from Sumitomo Seika Chemicals Co., Ltd., Osaka, Japan.

As described above with reference to Figs. 21-22, the absorbent core (i.e. the formed fibrous article 12) was formed to include an area 101 that was not been pin embossed, a first arcuate raised area 103, a second arcuate raised area 105, a pin embossed region 107 located between the raised area 103 and raised area 105, and a centrally extending pin embossed area 109 extending along the longitudinally extending central axis of the article 12, as shown in Fig. 21.

Area 101 had a thickness in the range of about 2 mm, a density of about 0.1 g/cc, and extended over a surface area of 6500 mm².

Area 103 and 105 each had a thickness of 5 mm and a density of about 0.05 g/cc. Each area 103 and 105 extended over a surface area of 557 mm².

Area 107 had a thickness of 0.2 mm and a density of 0.5 g/cc in those areas 111 embossed by the pins 78 and a density of 0.3 g/cc and a thickness of 1 mm in those areas outside of areas 111. Area 107 extended over a surface area of 504 mm².

Area 109 had a thickness of 0.2 mm and a density of 0.5 g/cc in those areas 111 embossed by the pins 78 and a thickness of 1 mm and a density of 0.3 g/cc in those areas outside of areas 111. Area 109 extended over a surface area of 4300 mm².

A barrier layer was arranged below the core and was formed from a 24 gsm polyethylene film commercially available from Clopay do Brasil, Sao Paulo, SP, Brazil.

Each of the layers of the sanitary napkin were adhered to one another using a conventional hot melt adhesive. After each of the layers were adhered to one another the sanitary napkin was passed through a conventional embossing process to form a raised area of the type shown in Fig. 23. Thus, the sanitary napkin included a planar portion 215 and a raised area 213 extending upwardly form the planar portion 215 as depicted in Fig. 23. The planar portion 215 extended over an area of 13000 mm² and the raised area 213 extended over an area of 2000 mm². The sanitary napkin had a thickness of 2.2 mm in the planar portion 215 and a thickness of 4.7 mm in the raised area 213.

### Inventive Example #2

An example of a sanitary napkin was constructed as follows. The body facing cover layer was constructed from a 27 gsm hot through air (HTA) bonded nonwoven material constructed from 100% bico fibers (PE/PET), commercially available from Shalag Industries A.C.S. Ltd., Kibbutz Shamir, Upper Galilee, Israel, under the commercial code STA4ETW27.

A 305 gsm formed fibrous absorbent core was arranged below the cover layer and was formed by the process described herein above with reference to Figs. 1-21.
The absorbent core had a composition of 89% by weight of pulp and 11% by weight of superabsorbent polymer. The pulp was Golden Isles Fluff Pulp 420#HD 7% Moisture, commercially available from GP Cellulose, Brunswick, Georgia, USA. The superabsorbent polymer was Aqua Keep SA70N commercially available from Sumitomo Seika Chemicals Co., Ltd., Osaka, Japan.

As described above with reference to Figs. 21-22, the absorbent core (i.e. the fibrous article 12) was formed to include an area 101 that was not been pin embossed, a first arcuate raised area 103, a second arcuate raised area 105, a pin embossed region 107 located between the raised area 103 and raised area 105, and a centrally extending pin embossed area 109 extending along the longitudinally extending central axis 13 of the article 12, as shown in Fig. 21.

Area 101 had a thickness in the range of about 3 mm, a density of about 0.09 g/cc, and extended over a surface area of 6500 mm².

Area 103 and 105 each had a thickness of 6 mm and a density of about 0.04 g/cc. Each area 103 and 105 extended over a surface area of 557 mm².

Area 107 had a thickness of 0.2 mm and a density of 0.5 g/cc in those areas 111 embossed by the pins 78 and a density of 0.3 g/cc and a thickness of 1 mm in those areas outside of areas 111. Area 107 extended over a surface area of 504 mm².

Area 109 had a thickness of 0.2 mm and a density of 0.5 g/cc in those areas 111 embossed by the pins 78 and a thickness of 1 mm and a density of 0.3 g/cc in those areas outside of areas 111. Area 109 extended over a surface area of 4300 mm².

Each of the layers of the sanitary napkin were adhered to one another using a conventional hot melt adhesive. After each of the layers were adhered to one another the sanitary napkin was passed through a conventional embossing process to form a raised area of the type shown in Fig. 23. Thus, the sanitary napkin included a planar portion 215 and a raised area 213 extending upwardly form the planar portion 215 as depicted in Fig. 23. The planar portion 215 extended over an area of 13000 mm² and the raised area 213 extended over an area of 2000 mm². The sanitary napkin had a thickness of 3.3 mm in the planar portion 215 and a thickness of 5.8 mm in the raised area 213.

### Comparative Example #1

A comparative example, representative of the prior art, was construed as follows. The body facing cover layer was constructed from a 27 gsm hot through air (HTA) bonded nonwoven material constructed from 100% bico fibers (PE/PET), commercially available from Shalag Industries A.C.S. Ltd., Kibbutz Shamir, Upper Galilee, Israel, under the commercial code STA4ETW27.

A 305 gsm fibrous absorbent core was arranged below the cover layer.
The absorbent core had a composition of 89% by weight of pulp and 11% by weight of superabsorbent polymer. The pulp was Golden Isles Fluff Pulp 420#HD 7% Moisture, commercially available from GP Cellulose, Brunswick, Georgia, USA. The superabsorbent polymer was Aqua Keep SA70N commercially available from Sumitomo Seika Chemicals Co., Ltd., Osaka, Japan. The absorbent core has a uniform thickness along its length of 2.2 mm and a uniform density along its length of 0.1 g/cc.

### Comparative Example #2

A comparative example, representative of the prior art, was construed as follows. The body facing cover layer was constructed from a 27 gsm hot through air (HTA) bonded nonwoven material constructed from 100% bico fibers (PE/PET), commercially available from Shalag Industries A.C.S. Ltd., Kibbutz Shamir, Upper Galilee, Israel, under the commercial code STA4ETW27.

A 305 gsm fibrous absorbent core was arranged below the cover layer,
The absorbent core had a composition of 89% by weight of pulp and 11% by weight of superabsorbent polymer. The pulp was Golden Isles Fluff Pulp 420#HD 7% Moisture, commercially available from GP Cellulose, Brunswick, Georgia, USA. The superabsorbent polymer was Aqua Keep SA70N commercially available from Sumitomo Seika Chemicals Co., Ltd., Osaka, Japan. The absorbent core has a uniform thickness along its length of 3.3 mm and a uniform density along its length of 0.09 g/cc.

### Test Procedures

Absorbent articles according to the present disclosure provide superior fluid handling characteristics. A number of test procedures are described below that highlight the fluid handling properties of absorbent articles according to the present disclosure. Prior to conducting any of the described test procedures described below the test product samples should be conditioned for two hours at 21 +/- 1° C and 50 +/- 2 % humidity.

### Procedure for Measuring Fluid Penetration Time

Fluid Penetration Time is measured by placing a product sample to be tested under a Fluid Penetration Test orifice plate. The orifice plate consists of a 7.6 cm X 25.4 cm plate of 1.3 cm thick polycarbonate with an elliptical orifice in its center. The elliptical orifice measures 3.8 cm along its major axis and 1.9 cm along its minor axis. The orifice plate is arranged such that the center of the orifice is aligned with the intersection of the longitudinal and transverse axis of the article, i.e. at the center of the article.

Test fluid was made of the following mixture to simulate bodily fluids:
49.5% of 0.9% sodium chloride solution (VWR catalog # VW 3257-7), 49.05% Glycerin (Emery 917), 1% Phenoxyethanol (Clariant Corporation Phenoxetol™) and 0.45% Sodium Chloride (Baker sodium chloride crystal # 9624-05).

A graduated 10 cc syringe containing 7 ml of test fluid is held over the orifice plate such that the exit of the syringe is approximately 3 inches above the orifice. The syringe is held horizontally, parallel to the surface of the test plate. The fluid is then expelled from the syringe at a rate that allows the fluid to flow in a stream vertical to the test plate into the orifice and a stop watch is started when the fluid first touches the sampie to be tested. The stop watch is stopped when a portion of the surface of the sample first becomes visible above the remaining fluid within the orifice. The elapsed time on the stop watch is the Fluid Penetration Time. The average Fluid Penetration Time (FPT) is calculated from taking the average of readings from three product samples.

### Procedure for Measuring Rewet Potential

The three product samples used for the Fluid Penetration Time (FPT) procedure described above are used for the Rewet Potential test described below.

The rewet potential is a measure of the ability of a napkin or other article to hold liquid within its structure when the napkin contains a relatively large quantity of liquid and is subjected to external mechanical pressure. The rewet potential is determined and defined by the following procedure.

The apparatus for the Rewet Potential test is the same as that set forth above with regard to the FPT test and further includes a quantity of 3 inch X 4 (7.62 cm X 10.16 cm) inch rectangles of Whatman #1 filter paper from (Whatman Inc., Clifton, NJ) and a weighing machine or balance capable of weighing to an accuracy of +/-.0.001 g, a quantity of said Whatman paper, a standard weight of 2.22 kg (4.8 pounds) having dimensions 5.1 cm (2 inches) by 10.2 cm (4.0 inches) by approximately 5.4 cm (2.13 inches) which applies a pressure of 4.14 kPa (0.6 psi) over the 5.1 by 10.2 cm (2 inches by 4 inches) surface.

For purposes of the test procedure set forth herein, the same three product samples used for the fluid penetration test should be used for the rewet potential test. After the test fluid is applied within the orifice plate in the FPT test described above, and as soon as the cover layer of the napkin first appears through the top surface of the fluid, the stop watch is started and an interval of 5 minutes is measured.

After 5 minutes have elapsed, the orifice plate is removed and the napkin is positioned on a hard level surface with the cover layer facing upwards.

A fifteen (15) layer stack of the pre-weighed filter paper is placed on and centered over the wetted area and the standard 2.22 kg weight is placed on top of the filter paper. The filter paper and the weight are arranged over the absorbent article such that they are centered over the area to which the fluid was applied. The filter paper and the weight are arranged such that their longer dimensions are aligned with the longitudinal direction of the product. Immediately after placing the paper and weight on the product, the stopwatch is started and after a 3 minute interval has elapsed the standard weight and filter paper are quickly removed. The wet weight of the filter paper is measured and recorded to the nearest 0.001 grams. The rewet value is then calculated as the difference in grams between the weight of the wet 15 layers of filter paper and the dry 15 layers of filter paper. The average Rewet Potential is calculated from taking the average of readings from three product samples.

### Procedure for Measuring the Thickness of a Sanitary Article

The apparatus required to measure the thickness of the sanitary napkin is a footed dial (thickness) gauge with stand, available from Ames, with a 2" (5.08 cm) diameter foot at a pressure of 0.07 psig (4.826 hPa) and a readout accurate to 0.001" (.0254 mm). A digital type apparatus is preferred. If the sanitary napkin sample is individually folded and wrapped, the sample is unwrapped and carefully flattened by hand. The release paper is removed from the product sample and it is repositioned back gently across the positioning adhesive lines so as not to compress the sample, ensuring that the release paper lies flat across the sample. Flaps (if any) are not considered when taking the thickness reading.

The foot of the gauge is raised and the product sample is placed on the anvil such that the foot of the gauge is approximately centered on the location of interest on the product sample. When lowering the foot, care must be taken to prevent the foot dropping onto the product sample or undue force being applied. A load of 4.826 hPa (0.07 psig) is applied to the sample and the read out is allowed to stabilize for approximately 5 seconds. The thickness reading is then taken. This procedure is repeated for three product samples and the average thickness is then calculated.

The measured Fluid Penetration Time, Rewet Potential and Product Thickness of the Inventive Examples and Comparative Examples described above are summarized in the table set forth below.

| | Thickness in Raised Area (mm) | Thickness in Planar Portion (mm) | Fluid Penetration Time (s) | Rewet (g) |
|---|---|---|---|---|
| Inventive Example #1 | 4.7 | 2.2 | 17 | 0.98 |
| Inventive Example #2 | 5.8 | 3.3 | 11 | 0.96 |
| Comparative Example #1 | NA | 2.2 | 52 | 0.82 |
| Comparative Example #2 | NA | 3.3 | 25 | 1.43 |

As shown above, disposable absorbent articles according to the present disclosure provide superior fluid handling characteristics.

## Claims

1. A method of making a formed fibrous article (12) comprising the steps of:
forming a formed fibrous article (12) having a three dimensional profile by forming a formed fibrous article (12) and passing the formed fibrous article (12) through a calendering station (16) to thereby provide the formed fibrous article (12) with the three dimensional profile; wherein the calendering station (16) includes a vacuum roll (42) and an opposed calender roll (44) and a surface of the calender roll includes at least one recess (53), the recess (53) being structured and arranged to provide the formed fibrous article (12) with the three dimensional profile;
pin-calendering the fibrous article (12) in a pin calendering station (18) between a first roll and a second roll, wherein the fibrous article (12) is conveyed through a nip defined between the first roll and the second roll without the use of a carrier layer;
wherein the first roll of the pin calendering station (18) comprises a vacuum roll (64) and the second roll comprises a pin calender roll (65).

2. An apparatus for making a formed fibrous article (12) having a three dimensional profile, the apparatus comprising:
a calendering station (16) including a first roll and a second roll, wherein the second roll includes at least one recess (53) for providing a formed fibrous article (12) with a three dimensional profile;
a pin calendering station (18) comprising a first roll and a second roll, wherein the first roll and second roll are structured and arranged to convey a fibrous article through a nip (92) defined between the first and second roll without the use of a carrier layer;
wherein the first roll of the pin calendering station (18) comprises a vacuum roll (64) and the second roll comprises a pin calender roll (65).

3. The method according to claim 1, wherein the vacuum roll (64) of the pin calendering station (18) includes a rotatable cylinder (66) and a plurality of holes (70) extending through a surface of the cylinder (66) and the pin calender roll (65) includes a plurality of pins (78) extending outwardly from a surface (80) of the roll (65).

4. The method according to claim 3, wherein each one of the plurality of pins (78) is arranged such that it does not overlap with any of the plurality of holes (70).

5. The method according claim 1, 3 or 4, wherein the formed fibrous article (12) having a three dimensional profile includes a planar portion (59) and at least one raised portion (61) extending upwardly relative to the planar portion (59);
wherein preferably the planar portion (59) and the raised portion (61) each have a different thickness and a different density but have a constant basis weight.

6. The method according to claim 1 or any one of claims 3 to 5, wherein the step of forming the fibrous article (12) comprises the steps of:
providing fibrous pulp (28), maintaining the fibrous pulp (28) in a chamber (30), mounting a mold (36) to a rotating forming drum (14), rotating the mold (36) on the rotating forming drum (14) until the mold (36) is arranged in communication with the chamber (30), and drawing the fibrous pulp (28) into the mold (36) to thereby form the fibrous article (12).

7. The method according to claim 1 or any one of claims 3 to 6, wherein the pin calender roll (65) includes a first area having a plurality the pins (78) extending outwardly from the surface (80) of the roll (65), a first recess (77), a second recess (79), a land area (81) arranged between the first (77) and second (79) recess, the land area (81) including a plurality of the pins (78) extending outwardly from the surface (80) of the roll (65).

8. The method according to claim 7, wherein the first recess (77), second recess (79) and land area (81) are structured and arranged to correspond in location to the raised portion (61) of the formed fibrous article (12) when the formed fibrous article (12) passes through the pin calendering station (18).

9. The method according to any one of claims 6 to 8, wherein the fibrous article (12) is formed in the mold (36) and transferred to the calendering station (16) without the use of a carrier layer.

10. The method according to claim 9, further comprising:
transferring the fibrous article (12) from the vacuum roll (42) of the calendering station (16) to the vacuum roll (64) of the pin calendering station (18).

11. The method according to claim 10, further comprising transferring the fibrous article (12) from the vacuum wheel (64) of the pin calendering station to a transfer wheel (20).

12. The method according to claim 11, wherein the transfer wheel (20) includes a vacuum roll (94) and a porous conveyor belt (97) that extends around the vacuum roll (94).

13. The apparatus according to claim 2, wherein the vacuum roll (64) of the pin calendering station (18) includes a rotatable cylinder (66) and a plurality of holes (70) extending through a surface of the cylinder (66) and the pin calender roll (65) includes a plurality of pins (78) extending outwardly from a surface (80) of the roll (65).

14. The apparatus according to claim 13, wherein each one of the plurality of pins (78) is arranged such that it does not overlap with any of the plurality of holes (70).

15. The apparatus according to any one of claims 2, 13 or 14, wherein the first roll of the calendering station (16) comprises a vacuum roll (42) and the second roll comprises an opposed calender roll (44).

16. The apparatus according to any one of claims 2, 13, 14 or 15, wherein the recess (53) extends over an area in the range of about 500 mm² to about 5000 mm².

17. The apparatus according to any one of claims 2, or 13 to 16, wherein the recess (53) has a depth in the range of about 2 mm to 25 mm as measured from a surface (47) of the roll (44) located outside of the recess (53).

18. The apparatus according to any one of claims 2 or 13 to 17, wherein the recess (53) has a maximum width in the range of about 20 mm to 120 mm as measured in a machine direction and a maximum width as measured in a transverse direction in the range of about 5 mm to about 60 mm.

19. The apparatus according to any one of claims 13 to 18, wherein the pin calender roll (64) includes a first area (69) having a plurality of the plurality of pins (78), the first area extending down a central portion of the roll surface (80).

20. The apparatus according to any one of claims 13 to 19, wherein the pin calender roll (64) includes a first recess (77), a second recess (79), and a land area (81) located between the first (77) and second (79) recess, the land area (81) including a plurality of the plurality of pins (78).

21. The apparatus according to any one of claims 2, or 13 to 20, further comprising a chamber (30) for holding fibrous pulp (28).

22. The apparatus according to claim 21, further comprising a forming drum (14) in communication with the chamber (30), wherein the forming drum (14) including a rotatable cylinder (15) having a plurality of molds (36) mounted thereto.

23. The apparatus according to claim 22, further comprising a vacuum chamber (38) arranged within the rotatable cylinder (15) of said forming drum (14), the vacuum chamber (38) being structured and arranged to sequentially draw the fibrous pulp (28) into each one of the plurality of molds (36).

24. The apparatus according to claim 23, wherein the vacuum roll (42) of the calendering station (16) includes a rotatable cylinder (46) having a plurality of holes (50) extending through a surface of the rotatable cylinder (46).

25. The apparatus according to claim 24, further comprising a vacuum chamber (56) arranged within the rotatable cylinder (46) of the calendering station (16), the vacuum chamber (46) adapted to draw air through the plurality of holes (50) in the rotatable cylinder (46).

26. The apparatus according to claim 25, wherein a leading edge of the vacuum chamber (56) arranged within the rotatable cylinder (46) of the calendering station (16) is substantially aligned with a trailing edge of the vacuum chamber (38) located with the rotatable cylinder (15) of the forming drum (14), the arrangement of the vacuum chambers (56;38) being adapted to transfer the fibrous article (12) from the forming drum (14) to the vacuum roll (42) of the calendering station (16).

27. The apparatus according to claim 26, wherein the vacuum roll (64) of the pin calendering station (18) includes a vacuum chamber (86) arranged within the rotatable cylinder (66) of the vacuum roll (64).

28. The apparatus according to claim 27, wherein the vacuum chamber (86) arranged within the rotatable cylinder (66) of the pin calendering station (18) is arranged so that a leading edge of the vacuum chamber (86) is substantially aligned with a trailing edge of the vacuum chamber (56) located within the rotatable cylinder (46) of the vacuum roll (42) in the calendering station (16).

29. The apparatus according to any one of claims 13 to 28, further comprising a transfer wheel (20).

30. The apparatus according to claim 29, wherein the transfer wheel (20) comprises a vacuum roll (94) and a porous conveyor belt (97).

31. The apparatus according to clam 30, wherein the vacuum roll (94) of the transfer wheel (20) includes a rotatable cylinder (96) and a vacuum chamber (106) arranged within the rotatable cylinder (96);
wherein preferably the porous conveyor belt (97) extends around the rotatable cylinder (96) of the transfer wheel (20).

32. The apparatus according to claim 31, wherein the vacuum chamber (106) arranged within the rotatable cylinder (96) of the transfer wheel (20) is arranged so that a leading edge of the vacuum chamber (106) is substantially aligned with a trailing edge of the vacuum chamber (86) located within the rotatable cylinder (66) of vacuum roll (64) in the pin calendering station (18).

## Patentansprüche

1. Verfahren zur Herstellung eines geformten faserigen Artikels (12), aufweisend die Schritte:
Bilden eines geformten faserigen Artikels (12) mit einem dreidimensionalen Profil durch Bilden eines geformten faserigen Artikels (12) und Durchgehen des geformten faserigen Artikels (12) durch eine Kalandrierungsstation (16), um dadurch den geformten faserigen Artikel (12) mit dem dreidimensionalen Profil bereitzustellen; wobei die Kalandrierungsstation (16) eine Vakuumwalze (42) und eine entgegengesetzte Kalanderwalze (44) enthält und eine Oberfläche der Kalanderwalze zumindest eine Vertiefung (53) enthält, wobei die Vertiefung (53) strukturiert und angeordnet ist, den geformten faserigen Artikel (12) mit dem dreidimensionalen Profil bereitzustellen;
Stiftkalandrieren des faserigen Artikels (12) in einer Stiftkalandrierungsstation (18) zwischen einer ersten Walze und einer zweiten Walze, wobei der faserige Artikel (12) durch einen Walzenspalt, der zwischen der ersten Walze und der zweiten Walze definiert ist, ohne Verwendung einer Trägerschicht, befördert wird;
wobei die erste Walze der Stiftkalandrierungsstation (18) eine Vakuumwalze (64) aufweist und die zweite Walze eine Stiftkalanderwalze (65) aufweist.

2. Vorrichtung zur Herstellung eines geformten faserigen Artikels (12) mit dreidimensionalem Profil, wobei die Vorrichtung aufweist:
eine Kalandrierungsstation (16), die eine erste Walze und eine zweite Walze enthält, wobei die zweite Walze zumindest eine Vertiefung (53) zum Bereitstellen eines geformten faserigen Artikels (12) mit dreidimensionalem Profil enthält;
eine Stiftkalandrierungsstation (18), aufweisend eine erste Walze und eine zweite Walze, wobei die erste Walze und die zweite Walze strukturiert und angeordnet sind, einen faserigen Artikel durch einen Walzenspalt (92), der zwischen der ersten und zweiten Walze definiert ist, ohne Verwendung einer Trägerschicht zu befördern;
wobei die erste Walze der Stiftkalandrierungsstation (18) eine Vakuumwalze (64) aufweist und die zweite Walze eine Stiftkalanderwalze (65) aufweist.

3. Verfahren nach Anspruch 1, wobei die Vakuumwalze (64) der Stiftkalandrierungsstation (18) einen drehbaren Zylinder (66) und mehrere Löcher (70), die sich durch eine Oberfläche des Zylinders (66) erstrecken, enthält, und die Stiftkalanderwalze (65) mehrere Stifte (78) enthält, die sich von einer Oberfläche (80) der Walze (65) nach außen erstrecken.

4. Verfahren nach Anspruch 3, wobei jeder der mehreren Stifte (78) so angeordnet ist, dass er nicht mit einem der mehreren Löcher (70) überlappt.

5. Verfahren nach Anspruch 1, 3 oder 4, wobei der geformte faserige Artikel (12) mit dreidimensionalem Profil einen ebenen Abschnitt (59) enthält und zumindest einen erhabenen Abschnitt (61), der sich relativ zum ebenen Abschnitt (59) nach oben erstreckt;
wobei bevorzugt der ebene Abschnitt (59) und der erhabene Abschnitt (61) jeweils eine unterschiedliche Dicke und eine unterschiedliche Dichte, aber ein konstantes Basisgewicht haben.

6. Verfahren nach Anspruch 1 oder einem der Ansprüche 3 bis 5, wobei der Schritt des Bildens des faserigen Artikels (12) die Schritte aufweist:
Bereitstellen eines Faserbreis (28), Halten des Faserbreis (28) in einer Kammer (30), Montieren einer Form (36) an eine drehende Formungstrommel (14), Drehen der Form (36) auf der drehenden Formungstrommel (14) bis die Form (36) in Verbindung mit der Kammer (30) angeordnet ist, und Ziehen des Faserbreis (28) in die Form (36), um dadurch den faserigen Artikel (12) zu bilden.

7. Verfahren nach Anspruch 1 oder einem der Ansprüche 3 bis 6, wobei die Stiftkalanderwalze (65) eine erste Fläche mit mehreren der Stifte (78), die sich von der Oberfläche (80) der Walze (65) nach außen erstrecken, einer ersten Vertiefung (77), einer zweiten Vertiefung (79), einem Stegbereich (81), der zwischen der ersten (77) und zweiten (79) Vertiefung angeordnet ist, enthält, wobei der Stegbereich (81) mehrere der Stifte (78), die sich von der Oberfläche (80) der Walze (65) nach außen erstrecken, enthält.

8. Verfahren nach Anspruch 7, wobei die erste Vertiefung (77), zweite Vertiefung (79) und der Stegbereich (81) strukturiert und angeordnet sind, um in Position dem erhabenen Abschnitt (61) des geformten faserigen Artikels (12) zu entsprechen, wenn der geformte faserige Artikel (12) durch die Stiftkalandrierungsstation (18) durchgeht.

9. Verfahren nach einem der Ansprüche 6 bis 8, wobei der faserige Artikel (12) in der Form (36) geformt wird und ohne Verwendung einer Trägerschicht zur Kalandrierungsstation (16) übertragen wird.

10. Verfahren nach Anspruch 9, des Weiteren aufweisend:
Übertragen des faserigen Artikels (12) von der Vakuumwalze (42) der Kalandrierungsstation (16) zur Vakuumwalze (64) der Stiftkalandrierungsstation (18).

11. Verfahren nach Anspruch 10, des Weiteren aufweisend ein Übertragen des faserigen Artikels (12) vom Vakuumrad (64) der Stiftkalandrierungsstation zu einem Übertragungsrad (20).

12. Verfahren nach Anspruch 11, wobei das Übertragungsrad (20) eine Vakuumwalze (94) und ein poröses Förderband (97) enthält, das sich um die Vakuumwalze (94) erstreckt.

13. Vorrichtung nach Anspruch 2, wobei die Vakuumwalze (64) der Stiftkalandrierungsstation (18) einen drehbaren Zylinder (66) und mehrere Löcher (70), die sich durch eine Oberfläche des Zylinders (66) erstrecken, enthält, und die Stiftkalanderwalze (65) mehrere Stifte (78) enthält, die sich von einer Oberfläche (80) der Walze (65) nach außen erstrecken.

14. Vorrichtung nach Anspruch 13, wobei jeder der mehreren Stifte (78) so angeordnet ist, dass er nicht mit einem der mehreren Löcher (70) überlappt.

15. Vorrichtung nach einem der Ansprüche 2, 13 oder 14, wobei die erste Walze der Kalandrierungsstation (16) eine Vakuumwalze (42) aufweist und die zweite Walze eine entgegengesetzte Kalanderwalze (44) aufweist.

16. Vorrichtung nach einem der Ansprüche 2, 13, 14 oder 15, wobei sich die Vertiefung (53) über eine Fläche im Bereich von ungefähr 500 mm² bis ungefähr 5000 mm² erstreckt.

17. Vorrichtung nach einem der Ansprüche 2, oder 13 bis 16, wobei die Vertiefung (53) eine Tiefe im Bereich von ungefähr 2 mm bis 25 mm hat, gemessen von einer Oberfläche (47) der Walze (44), die außerhalb der Vertiefung (53) gelegen ist.

18. Vorrichtung nach einem der Ansprüche 2 oder 13 bis 17, wobei die Vertiefung (53) eine maximale Breite im Bereich von ungefähr 20 mm bis 120 mm, gemessen in einer Maschinenrichtung, und eine maximale Breite, gemessen in einer querlaufenden Richtung, im Bereich von ungefähr 5 mm bis ungefähr 60 mm hat.

19. Vorrichtung nach einem der Ansprüche 13 bis 18, wobei die Stiftkalanderwalze (64) eine erste Fläche (69) mit mehreren der mehreren Stifte (78) enthält, wobei sich der erste Bereich entlang eines Mittelabschnitts der Walzenoberfläche (80) nach unten erstreckt.

20. Vorrichtung nach einem der Ansprüche 13 bis 19, wobei die Stiftkalanderwalze (64) eine erste Vertiefung (77), eine zweite Vertiefung (79) und einen Stegbereich (81), der zwischen der ersten (77) und zweiten (79) Vertiefung positioniert ist, enthält, wobei der Stegbereich (81) mehrere der mehreren Stifte (78) enthält.

21. Vorrichtung nach einem der Ansprüche 2, oder 13 bis 20, des Weiteren aufweisend eine Kammer (30) zum Halten des Faserbreis (28).

22. Vorrichtung nach Anspruch 21, des Weiteren aufweisend eine Formungstrommel (14) in Verbindung mit der Kammer (30), wobei die Formungstrommel (14) einen drehbaren Zylinder (15) mit mehreren darauf montierten Formen (36) enthält.

23. Vorrichtung nach Anspruch 22, des Weiteren aufweisend eine Vakuumkammer (38), die innerhalb des drehbaren Zylinders (15) der Formungstrommel (14) angeordnet ist, wobei die Vakuumkammer (38) strukturiert und angeordnet ist, den Faserbrei (28) sequenziell in jede der mehreren Formen (36) zu ziehen.

24. Vorrichtung nach Anspruch 23, wobei die Vakuumwalze (42) der Kalandrierungsstation (16) einen drehbaren Zylinder (46) mit mehreren Löchern (50), die sich durch eine Oberfläche des drehbaren Zylinders (46) erstrecken, enthält.

25. Vorrichtung nach Anspruch 24, des Weiteren aufweisend eine Vakuumkammer (56), die innerhalb des drehbaren Zylinders (46) der Kalandrierungsstation (16) angeordnet ist, wobei die Vakuumkammer (46) angepasst ist, Luft durch die mehreren Löcher (50) in den drehbaren Zylinder (46) zu ziehen.

26. Vorrichtung nach Anspruch 25, wobei eine Führungskante der Vakuumkammer (56), die innerhalb des drehbaren Zylinders (46) der Kalandrierungsstation (16) angeordnet ist, im Wesentlichen mit einer Hinterkante der Vakuumkammer (38), die am drehbaren Zylinder (15) der Formungstrommel (14) gelegen ist, ausgerichtet ist, wobei die Anordnung der Vakuumkammern (56; 38) angepasst ist, den faserigen Artikel (12) von der Formungstrommel (14) zur Vakuumwalze (42) der Kalandrierungsstation (16) zu übertragen.

27. Vorrichtung nach Anspruch 26, wobei die Vakuumwalze (64) der Stiftkalandrierungsstation (18) eine Vakuumkammer (86) enthält, die innerhalb des drehbaren Zylinders (66) der Vakuumwalze (64) angeordnet ist.

28. Vorrichtung nach Anspruch 27, wobei die Vakuumkammer (86), die innerhalb des drehbaren Zylinders (66) der Stiftkalandrierungsstation (18) angeordnet ist, so angeordnet ist, dass eine Führungskante der Vakuumkammer (86) im Wesentlichen mit einer Hinterkante der Vakuumkammer (56) ausgerichtet ist, die innerhalb des drehbaren Zylinders (46) der Vakuumwalze (42) in der Kalandrierungsstation (16) gelegen ist.

29. Vorrichtung nach einem der Ansprüche 13 bis 28, des Weiteren aufweisend ein Übertragungsrad (20).

30. Vorrichtung nach Anspruch 29, wobei das Übertragungsrad (20) eine Vakuumwalze (94) und ein poröses Förderband (97) aufweist.

31. Vorrichtung nach Anspruch 30, wobei die Vakuumwalze (94) des Übertragungsrads (20) einen drehbaren Zylinder (96) und eine Vakuumkammer (106), die innerhalb des drehbaren Zylinders (96) angeordnet ist, enthält;
wobei sich das poröse Förderband (97) vorzugsweise um den drehbaren Zylinder (96) des Übertragungsrads (20) erstreckt.

32. Vorrichtung nach Anspruch 31, wobei die Vakuumkammer (106), die innerhalb des drehbaren Zylinders (96) des Übertragungsrads (20) angeordnet ist, so angeordnet ist, dass eine Führungskante der Vakuumkammer (106) im Wesentlichen mit einer Hinterkante der Vakuumkammer (86) ausgerichtet ist, die innerhalb des drehbaren Zylinders (66) der Vakuumwalze (64) in der Stiftkalandrierungsstation (18) gelegen ist.

## Revendications

1. Procédé de fabrication d'un article fibreux formé (12), comprenant les étapes suivantes :
former un article fibreux formé (12) ayant un profil tridimensionnel en formant un article fibreux formé (12) et en faisant passer l'article fibreux formé (12) à travers un poste de calandrage (16) pour ainsi fournir à l'article fibreux formé (12) son profil tridimensionnel ; le poste de calandrage (16) comportant un rouleau aspirant (42) et un rouleau de calandre opposé (44) et une surface du rouleau de calandre comportant au moins un renfoncement (53), le renfoncement (53) étant structuré et agencé de manière à fournir à l'article fibreux formé (12) son profil tridimensionnel ;
effectuer un calandrage à broche de l'article fibreux (12) dans un poste de calandrage à broche (18) entre un premier rouleau et un deuxième rouleau, l'article fibreux (12) étant transporté à travers une fente définie entre le premier rouleau et le deuxième rouleau sans utiliser de couche porteuse ;
le premier rouleau du piston de calandrage à broche (18) comprenant un rouleau aspirant (64) et le deuxième rouleau comprenant un rouleau de calandrage à broche (65).

2. Appareil de fabrication d'un article fibreux formé (12) ayant un profil tridimensionnel, l'appareil comprenant :
un poste de calandrage (16) comportant un premier rouleau et un deuxième rouleau, le deuxième rouleau comportant au moins un renfoncement (53) pour fournir à un article fibreux formé (12) un profil tridimensionnel ;
un poste de calandrage à broche (18) comprend un premier rouleau et un deuxième rouleau, le premier rouleau et le deuxième rouleau étant structurés et agencés de manière à transporter un article fibreux à travers une fente (92) définie entre le premier et le deuxième rouleau sans utiliser de couche porteuse ;
le premier rouleau du poste de calandrage à broche (18) comprenant un rouleau aspirant (64) et le deuxième rouleau comprenant un rouleau de calandrage à broche (65).

3. Procédé selon la revendication 1, dans lequel le rouleau aspirant (64) du poste de calandrage à broche (18) comporte un cylindre rotatif (66) et une pluralité de trous (70) s'étendant à travers une surface du cylindre (66) et le rouleau de calandrage à broche (65) comporte une pluralité de broches (78) s'étendant vers l'extérieur depuis une surface (80) du rouleau (65).

4. Procédé selon la revendication 3, dans lequel chacune de la pluralité de broches (78) est disposée de manière à ne pas recouvrir l'un quelconque parmi la pluralité de trous (70).

5. Procédé selon la revendication 1, 3 ou 4, dans lequel l'article fibreux formé (12) ayant un profil tridimensionnel comporte une portion plane (59) et au moins une portion rehaussée (61) s'étendant vers le haut par rapport à la portion plane (59) ;
la portion plane (59) et la portion rehaussée (61) ayant chacune une épaisseur différente et une densité différente mais ayant un poids de base constant.

6. Procédé selon la revendication 1 ou selon l'une quelconque des revendications 3 à 5, dans lequel l'étape de formage de l'article fibreux (12) comprend les étapes suivantes :
fournir une pâte fibreuse (28), maintenir la pâte fibreuse (28) dans une chambre (30), monter un moule (36) sur un tambour de formage rotatif (14), faire tourner le moule (36) sur le tambour de formage rotatif (14) jusqu'à ce que le moule (36) soit agencé en communication avec la chambre (30), et tirer la pâte fibreuse (28) dans le moule (36) pour ainsi former l'article fibreux (12).

7. Procédé selon la revendication 1 ou selon l'une quelconque des revendications 3 à 6, dans lequel le rouleau de calandrage à broche (65) comporte une première zone ayant une pluralité de broches (78) s'étendant vers l'extérieur depuis la surface (80) du rouleau (65), un premier renfoncement (77), un deuxième renfoncement (79), une zone de méplat (81) agencée entre le premier renfoncement (77) et le deuxième renfoncement (79), la zone de méplat (81) comportant une pluralité de broches (78) s'étendant vers l'extérieur depuis la surface (80) du rouleau (65).

8. Procédé selon la revendication 7, dans lequel le premier renfoncement (77), le deuxième renfoncement (79) et la zone de méplat (81) sont structurés et agencés de manière à correspondre, de par leur positionnement, à la portion rehaussée (61) de l'article fibreux formé (12) lorsque l'article fibreux formé (12) passe à travers le poste de calandrage à broche (18).

9. Procédé selon l'une quelconque des revendications 6 à 8, dans lequel l'article fibreux (12) est formé dans le moule (36) et est transféré au poste de calandrage (16) sans utiliser de couche porteuse.

10. Procédé selon la revendication 9, comprenant en outre :
le transfert de l'article fibreux (12) depuis le rouleau aspirant (42) du poste de calandrage (16) jusqu'au rouleau aspirant (64) du poste de calandrage à broche (18).

11. Procédé selon la revendication 10, comprenant en outre le transfert de l'article fibreux (12) depuis la roue aspirante (64) du poste de calandrage à broche jusqu'à une roue de transfert (20).

12. Procédé selon la revendication 11, dans lequel la roue de transfert (20) comporte un rouleau aspirant (94) et une courroie transporteuse poreuse (97) qui s'étend autour du rouleau aspirant (94).

13. Appareil selon la revendication 2, dans lequel le rouleau aspirant (64) du poste de calandrage à broche (18) comporte un cylindre rotatif (66) et une pluralité de trous (70) s'étendant à travers une surface du cylindre (66) et le rouleau de calandrage à broche (65) comporte une pluralité de broches (78) s'étendant vers l'extérieur depuis une surface (80) du rouleau (65).

14. Appareil selon la revendication 13, dans lequel chacune de la pluralité de broches (78) est agencée de manière à ne pas recouvrir l'un quelconque de la pluralité de trous (70).

15. Appareil selon l'une quelconque des revendications 2, 13 ou 14, dans lequel le premier rouleau du poste de calandrage (16) comprend un rouleau aspirant (42) et le deuxième rouleau comprend un rouleau de calandre opposé (44).

16. Appareil selon l'une quelconque des revendications 2, 13, 14 ou 15, dans lequel le renfoncement (53) s'étend sur une zone dans une plage d'environ 500 mm² à environ 5000 mm².

17. Appareil selon l'une quelconque des revendications 2 ou 13 à 16, dans lequel le renfoncement (53) a une profondeur dans une plage d'environ 2 mm à 25 mm, mesurée à partir d'une surface (47) du rouleau (44) située à l'extérieur du renfoncement (53).

18. Appareil selon l'une quelconque des revendications 2 ou 13 à 17, dans lequel le renfoncement (53) a une largeur maximale de l'ordre d'environ 20 mm à 120 mm, mesurée dans un sens machine, et une largeur maximale mesurée dans un sens travers de l'ordre d'environ 5 mm à environ 60 mm.

19. Appareil selon l'une quelconque des revendications 13 à 18, dans lequel le rouleau de calandrage à broche (64) comporte une première zone (69) ayant une pluralité de la pluralité de broches (78), la première zone s'étendant le long d'une portion centrale de la surface de rouleau (80).

20. Appareil selon l'une quelconque des revendications 13 à 19, dans lequel le rouleau de calandre à broche (64) comporte un premier renfoncement (77), un deuxième renfoncement (79), une zone de méplat (81) située entre le premier renfoncement (77) et le deuxième renfoncement (79), la zone de méplat (81) comportant une pluralité de la pluralité de broches (78).

21. Appareil selon l'une quelconque des revendications 2 ou 13 à 20, comprenant en outre une chambre (30) pour retenir la pâte fibreuse (28).

22. Appareil selon la revendication 21, comprenant en outre un tambour de formage (14) en communication avec la chambre (30), le tambour de formage (14) comportant un cylindre rotatif (15) ayant une pluralité de moules (36) montés sur celui-ci.

23. Appareil selon la revendication 22, comprenant en outre une chambre à vide (38) agencée à l'intérieur du cylindre rotatif (15) dudit tambour de formage (14), la chambre à vide (38) étant structurée et agencée de manière à tirer successivement la pâte fibreuse (28) dans chacun de la pluralité de moules (26).

24. Appareil selon la revendication 23, dans lequel le rouleau aspirant (42) du poste de calandrage (16) comporte un cylindre rotatif (46) ayant une pluralité de trous (50) s'étendant à travers une surface du cylindre rotatif (46).

25. Appareil selon la revendication 24, comprenant en outre une chambre à vide (56) agencée à l'intérieur du cylindre rotatif (46) du poste de calandrage (16), la chambre à vide (46) étant prévue pour aspirer de l'air à travers la pluralité de trous (50) dans le cylindre rotatif (46).

26. Appareil selon la revendication 25, dans lequel un bord avant de la chambre à vide (56) agencé à l'intérieur du cylindre rotatif (46) du poste de calandrage (16) est substantiellement aligné avec un bord arrière de la chambre à vide (38) située à l'intérieur du cylindre rotatif (15) du tambour de formage (14), l'agencement des chambres à vide (56 ; 38) étant prévu pour transférer l'article fibreux (12) du tambour de formage (14) au rouleau aspirant (42) du poste de calandrage (16).

27. Appareil selon la revendication 26, dans lequel le rouleau aspirant (64) du poste de calandrage à broche (18) comporte une chambre à vide (86) agencée à l'intérieur du cylindre rotatif (66) du rouleau aspirant (64).

28. Appareil selon la revendication 27, dans lequel la chambre à vide (86) agencée à l'intérieur du cylindre rotatif (66) du poste de calandrage à broche (18) est agencée de manière à ce qu'un bord avant de la chambre à vide (86) soit substantiellement aligné avec un bord arrière de la chambre à vide (56) située à l'intérieur du cylindre rotatif (46) du rouleau aspirant (42) dans le poste de calandrage (16).

29. Appareil selon l'une quelconque des revendications 13 à 28, comprenant en outre une roue de transfert (20).

30. Appareil selon la revendication 29, dans lequel la roue de transfert (20) comprend un rouleau aspirant (94) et une courroie de transport poreuse (97).

31. Appareil selon la revendication 30, dans lequel le rouleau aspirant (94) de la roue de transfert (20) comporte un cylindre rotatif (96) et une chambre à vide (106) agencée à l'intérieur du cylindre rotatif (96) ;
la courroie transporteuse poreuse (97) s'étendant de préférence autour du cylindre rotatif (96) de la roue de transfert (20).

32. Appareil selon la revendication 31, dans lequel la chambre à vide (106) agencée à l'intérieur du cylindre rotatif (96) de la roue de transfert (20) est agencée de telle sorte qu'un bord avant de la chambre à vide (106) soit substantiellement aligné avec un bord arrière de la chambre à vide (86) située à l'intérieur du cylindre rotatif (66) du rouleau aspirant (64) dans le poste de calandrage à broche (18).
